# EUROPÄISCHE PATENTSCHRIFT

(11) **EP 2 464 717 B1**
(45) Veröffentlichungstag und Bekanntmachung des Hinweises auf die Patenterteilung: **26.12.2018**
(21) Anmeldenummer: 10740675.3
(22) Anmeldetag: 11.08.2010
(51) Int. Cl.: C12M 1/08, C12M 1/00

(54) **VORRICHTUNG UND VERFAHREN ZUR FERMENTATION**
DEVICE AND METHOD FOR FERMENTATION
DISPOSITIF ET PROCÉDÉ EN VUE DE LA FERMENTATION

(30) Priorität: 12.08.2009 DE 102009026366
(43) Veröffentlichungstag der Anmeldung: 20.06.2012
(73) Patentinhaber: Krones AG, 93073 Neutraubling (DE)
(72) Erfinder: MÜLLER-AUFFERMANN, Konrad, 85354 Freising (DE)
(74) Vertreter: Grünecker Patent- und Rechtsanwälte PartG mbB
(86) Internationale Anmeldenummer: PCT/EP2010/061656
(87) Internationale Veröffentlichungsnummer: WO 2011/018473

(56) Entgegenhaltungen:
- WO-A1-86/07604
- WO-A1-2007/087794
- DE-A1- 2 603 769
- DE-A1- 3 429 355
- DE-A1- 3 727 236
- GB-A- 1 515 221
- US-A- 5 654 197

## Beschreibung

Die vorliegende Erfindung betrifft einen Tank zur Fermentation. Der Tank umfasst mindestens eine Befüllleitung zur Zuführung einer frischen Flüssigkeit und mindestens eine Entnahmeleitung zur Ableitung einer zumindest teilweise fermentierten Flüssigkeit.

Die Erfindung betrifft weiterhin eine Vorrichtung zur Fermentation. Die Vorrichtung umfasst eine mehrstufige Anordnung von zwei oder mehreren, miteinander in einer Kaskade gekoppelten Tanks zur Fermentation.

Zudem betrifft die Erfindung ein Verfahren zur kontinuierlichen oder semikontinuierlichen Fermentation von Flüssigkeiten innerhalb mindestens eines Tanks zur Fermentation.

Es sind bereits zahlreiche unterschiedliche Verfahren und Vorrichtungen zur Fermentation von Flüssigkeiten (Substrat) bekannt, die überwiegend zur Lebensmittelherstellung eingesetzt werden, und die teilweise durch Modifikation herkömmlicher Fermenter mit Ein- und/oder Umbauten gebildet wurden.
Die DE 24 36 793 A1 offenbart beispielsweise eine Vorrichtung zur aeroben Fermentation eines Substrates durch Mikroorganismen, bei der in einem Fermenter ein nach unten und oben offenes Leitrohr mit einem zylindrischen Mantel koaxial angeordnet ist. Außerhalb des Fermenters befindet sich eine Umwälzpumpe mit zugehörigen Leitungen, welche die in den Fermenter einzubringende und/oder die im Fermenter vorhandene Flüssigkeit unter Druck fördert. Für einen kontinuierlichen Betrieb können auch frisches Nährmedium oder Kreislauffiltrat und Substrat mittels einer Düse oder mittels einer weiteren in den Fermenter führenden Leitung zugeführt werden, während ein Teil der Fermenterbrühe aus dem Fermenter direkt oder über einen Abzweig in der Druckleitung der Umwälzpumpe abgezogen wird. Der hier beschriebene Stand der Technik sieht keinerlei Möglichkeiten vor, sedimentierende Stoffe abzuscheiden, weil hier eine homogene Durchmischung des Behälterinhalts angestrebt wird. Zu diesem Zweck ist der ständige Betrieb einer Umwälzpumpe unabdinglich (= Zwangsumwälzung).

Aus der DE 38 10 843 A1 ist ein Pulsantrieb für volldurchmischte Bioreaktoren nach dem Schlaufenprinzip bekannt. Auch hier befindet sich ein nach oben und unten offenes Leitrohr innerhalb des Fermenters, das einer Durchmischung des Tankinhalts dient. Mittels einer in der Mittelachse des Leitrohrs angeordneten Düse soll hier eine Zirkulation eines in den Reaktorbehälter eingebrachten Substrats eingestellt werden. Der Reaktorkörper des Bioreaktors weist ebenfalls einen zylindrischen Mantel auf. Das beidseitig offene Leitrohr erstreckt sich über ca. 80% der nutzbaren Reaktorhöhe bis hin zu einem Siebboden und ist mittig, d.h. mit gleichem oberen und unteren Abstand zentrisch, im Reaktorkörper fixiert. In diesem mittleren Bereich des Reaktors erfolgt eine schlaufenförmige Umwälzung. Oberhalb des Siebbodens befindet sich zudem ein horizontal verlaufender Ablaufstutzen, während ein horizontal verlaufender Zulaufstutzen für das umzusetzende Substrat unterhalb des Siebbodens in den Abwärtsstrom des Kreislaufs führt. Die gewünschte Umwälzung des Reaktorinhaltes erfolgt hier durch den Betrieb des Pulsantriebs (= Zwangsströmung).

Die DE 34 29 355 A1 offenbart schließlich eine Vorrichtung und ein Verfahren zur Durchführung von verfahrenstechnischen Grundoperationen und biochemischen Reaktionen in Flüssig- oder Gas-Flüssig-Systemen mit Kaskaden bestehend aus mehreren in einem geschlossenen Reaktor befindlichen, hintereinander geschalteten Kammern, wobei Gas und/oder andere spezifisch leichtere Stoffe in Teilbereichen der Kammern aufsteigen und dadurch in anderen Teilbereichen der Kammern eine abwärts gerichtete Strömung der Flüssigkeit bewirken, die zu freien oder gelenkten Umlaufströmungen der Flüssigkeit in den Kammern führt, an denen auch fein dispergierte andere Stoffe teilnehmen können. Durch diese Strömungen soll ebenfalls eine bessere Durchmischung des Reaktorinhalts innerhalb der einzelnen Kammern erreicht werden.

Die Patentschrift DE 656 361 A offenbart eine Gärvorrichtung, die ein Gärgefäß und ein Steigrohr umfasst. In einem unteren Bereich des Steigrohrs wird Druckluft eingeführt. Das Steigrohr verjüngt sich von unten nach oben. Durch die Verjüngung erhöht sich die Steiggeschwindigkeit im Steigrohr. Auch hier wird eine vollständige Durchmischung der einzelnen Reaktorstufen angestrebt und die Partikel und Organismen verbleiben im System. Durch die notwendige Druckluftzufuhr wird eine Zwangsdurchströmung erreicht.

Die deutsche Patentanmeldung DE 37 27 236 A1 offenbart einen Festbett-Umlaufreaktor. Der Reaktorbehälter umfasst einen Behälterboden und ein zentrales Rohrsystem. Der Ringraum zwischen dem Rohrsystem und dem Behältermantel ist mit einer Trägermaterial-Schüttung versehen. Man erreicht somit eine hohe Konzentration der Biomasse im Trägermaterial, das porös ist. Auch hier erfolgt eine Zwangsdurchströmung.

Die deutsche Patentanmeldung DE 36 03 792 A1 offenbart einen mehrstufigen Reaktor zur Stoffumwandlung. In einem unteren Bereich des Reaktors wird die Flüssigkeit tangential zugeführt, so dass es zu einem guten Kontakt mit den Katalysatoren kommt. Durch Überstromkanäle wird die Flüssigkeit in die nächsten Stufen geleitet, deren Böden ebenfalls trichterförmig ausgebildet sind. Nach Erreichen der letzten Stufe wird die Flüssigkeit abgezogen.

Ziel des im dem Patent GB 1515221 beschriebenen Verfahrens ist, Gase, die aufgrund ihrer Dichte normalerweise in Flüssigkeiten aufsteigen, durch ein Gegenstromverfahren möglichst lange in Schwebe zu halten, um so eine gute Gaslösung zu gewähren. Dabei erfolgt die Flüssigkeits-/Gaszugabe durch mechanischen Aufwand via Pumpen (= Zwangsströmung). GB 1515221 dient dabei nicht der Fermentation, bei der üblicherweise Gase entstehen. Die internationale Patentanmeldung WO 86/07604 beschreibt Reaktortypen die derart ausgeführt sind, dass eine Zwangsumwälzung des Fermentates aufgrund der Einleitung eines Gases in einen unteren Tankbereich resultiert. Dabei gibt es in den Reaktoren, in denen aufgrund der Gaseinleitung eine turbulente Strömung vorherrscht, Sedimentationszonen in denen sich Organismen absetzen können, so dass diese lange im System verweilen. Ein Entfernen der Organismen in bestimmten Intervallen ist jedoch von hoher Wichtigkeit, da sich ansonsten Mutationen ergeben können, die unter Umständen zu starken sensorischen und chemisch-physikalischen Veränderungen führen.

Das US Patent 5,654,197 beschreibt Rührkesselreaktoren, die kontinuierlich betrieben werden. Um ein Zurückhalten der Organismen (bzw. der Trägermaterialien auf denen die Organismen fixiert sind) im System zu gewährleisten, sind Sedimentationszonen vorgesehen. Diese sind teilweise so ausgeführt, dass ein sich verjüngendes Entnahmerohr in die Flüssigkeit eintaucht. Auch hier sollen die im Fermentat enthaltenen Organismen möglichst lange und vollständig in jedem Reaktor belassen werden. Daher werden Sedimentationszonen vorgesehen, in denen eine Abscheidung erfolgt, wobei die sedimentierten Organismen wieder in die turbulente Zwangsumwälzung (impliziert durch das Rühren) geraten.

Die deutsche Offenlegungsschrift DE 26 03 769 A1 offenbart eine aerobe Fermentation nach dem oben beschriebenen Prinzip des Schlaufenreaktors (vgl. DE 24 36 793), wobei hier das nach oben und unten offene Strömungsleitrohr als Wärmeübertrager ausgebildet ist.

In der WO 2007/087794 A1 wird eine Vorrichtung zur Erzeugung von Biogas beschrieben, die einen Bioreaktor aufweist, der eine Einfüllkammer und einen Rücklaufkanal zum Abführen organischer Stoffe aus dem Bioreaktor umfasst.

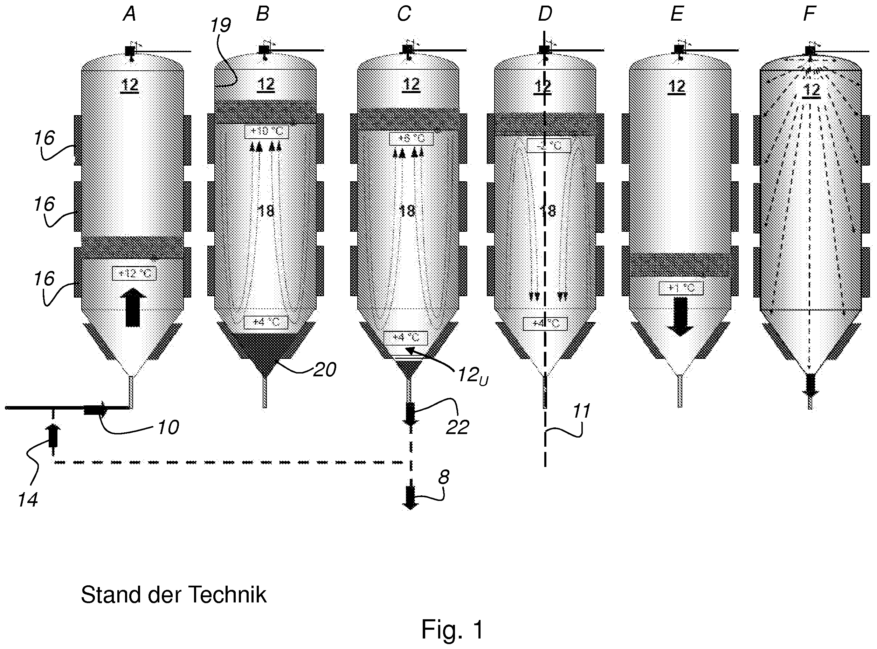

Die schematische Darstellung der Fig. 1 zeigt zunächst eine aus dem Stand der Technik bekannte Verfahrensfolge einer klassischen chargenweisen Fermentation am Beispiel der Bierherstellung. Dargestellt sind hier die aufeinander folgenden Prozessschritte anhand von sechs Verfahrensstadien A bis F. In einem ersten Verfahrensschritt A wird ein mit Organismen (Hefen) versetztes Substrat 10 (Basisfermentat: Bierwürze; Flüssigkeit) von unten in einen Tank 12 geleitet. Da die Tanks 12 für die Fermentation meist große Volumina aufweisen, dauert die Befüllung moderner Großtanks meist mehrere Stunden. Der erste Teil der Vergärung wird oftmals als Hauptgärung bezeichnet. In dieser Zeitspanne, die bei der Bierfermentation meist mehrere Tage dauert, wird der größte Teil des Substrats 10 umgewandelt bzw. verstoffwechselt. Um die bei der Gärung entstehende Wärme abzuführen und den Tankinhalt gezielt zu temperieren, sind die Tanks 12 meist mit verschiedenen Kühlzonen 16 ausgestattet. Durch die Temperierung über die Kühlzonen 16 entstehen im Tankinneren Konvektionsströmungen 18, wie sie vereinfacht in den Prozessschritten B, C und D angedeutet sind. Wie dargestellt, sinkt bei der Hauptgärung B und C das Fermentat 10 zunächst an der gekühlten Wandung 19 des Tanks 12 tendenziell ab, da kältere Medien bis zu einem gewissen Grad eine höhere Dichte aufweisen. Gleichzeitig sedimentieren Partikel und/oder Organismen und sammeln sich an einer am Tankboden eines unteren Bereichs 12_{U} ausgebildeten Konusspitze 20. Weil die Kühlung von außen, meist über die entsprechenden Kühlzonen 16 erfolgt, herrschen zur Mittelachse 11 des Tanks 12 hin höhere Temperaturen, so dass sich tendenziell ein natürlicher von unten nach oben aufsteigender Kernfluss der wärmeren Flüssigkeit ergibt; vgl. Prozessschritte B und C, unter Berücksichtigung der Dichteanomalie von Wasser. Nachdem eine gewünschte Substratumsetzung erfolgt ist, wird der Tankinhalt meistens intensiver abgekühlt, so dass die in der Flüssigkeit enthaltenen Hefen und Partikel verstärkt in den unteren Bereich 12_{U} des Tanks 12 absinken. Anschließend wird die in der Konusspitze 20 sedimentierte Hefe 22 vorsichtig abgezogen, als Inokulat 14 weiterverarbeitet oder verworfen (Bezugsziffer 8). Der restliche Tankinhalt wird dann entweder im gleichen Tank 12 zur Reifung und Lagerung belassen oder in spezielle Reifungs- und Lagertanks gepumpt. Die Verfahrensbeschreibung (sog. Eintank-, Zweitank- oder Dreitankverfahren) orientiert sich dabei an der Anzahl der zur Gärung verwendeten Tanks 12. Während der Reifung und Lagerung wird der Tankinhalt meistens auf sehr geringe Temperaturen abgekühlt, um weitere Ausfällungen und gewünschte chemisch-physikalische Reaktionen zu erreichen. Weil die Kaltreifung und Lagerung oft bei Temperaturen erfolgt, die unterhalb der für die Flüssigkeit größten Dichte liegen, kann sich während dieses Prozesses die Strömungsrichtung innerhalb des Tanks 12 umdrehen; vgl. Prozessbild D unter Berücksichtigung der Dichteanomalie von Wasser. Demnach sinken die dichtesten, jedoch nicht zwangsläufig die kältesten Flüssigkeitspakete nach unten. Obgleich die Flüssigkeit an der Wandung 19 des Tanks 12 stärker abgekühlt wird (z. B. auf -2 °C), steigt die kältere, jedoch weniger dichte Flüssigkeit jetzt tendenziell an der Wandung 19 nach oben. In der weniger kalten Tankmitte um die Mittelachse 11 erhöht sich nun die Dichte der Flüssigkeit und der Kernstrom fließt tendenziell von oben nach unten. Dabei ist jedoch immer zu beachten, dass während der voranschreitenden Fermentation im Substrat enthaltene Stoffe umgewandelt werden. Diese Stoffe, wie bspw. Zucker, Kohlenstoffdioxid und Alkohol, beeinflussen das Strömungssystem zusätzlich, da sich Dichteverschiebungen ergeben können. Nachdem der Reife- bzw. Lagerprozess abgeschlossen ist, folgt die mehrstündige Tankentleerung, welche im Prozessbild E dargestellt ist. Anschließend werden die Tanks 12 jeweils einem mehrstündigen Reinigungsintervall F unterzogen, bevor sie für die nächste Fermentation bereit stehen. Da während der alkoholischen Gärung auch Fermentationsgas (in diesem Fall Kohlenstoffdioxid) entsteht, sind die Tanks 12 meistens mit Armaturen ausgestattet, die eine Einstellung des Drucks ermöglichen und das überschüssige Gas ableiten. In modernen Betrieben wird das während der Gärung entstandene Gas aufgefangen und je nach Reinheitsgrad entsprechend aufgearbeitet und weiterverwendet. Wie aus der Darstellung der aufeinander folgenden Prozessschritte A - F deutlich wird, nimmt der eigentliche Gärprozess nur einen Teil der aufzuwendenden Gesamttankbelegungszeit ein, da die Befüllung, Entleerung und Reinigung der Tanks 12 ebenfalls einen relativ großen Zeitbedarf haben, nämlich jeweils mehrere Stunden. Dieser Aufwand wird bei Mehrtankverfahren (Zwei- oder Dreitankverfahren), in denen die Hauptgärung, Reifung und/oder die Lagerung in unterschiedlichen Tanks 12 erfolgt, zusätzlich erhöht. Sobald diese Zeiten einen zu großen Anteil der gesamten Tankbelegung ausmachen, werden die Verfahrensökonomie und damit die Wirtschaftlichkeit des Fermentationsverfahrens drastisch reduziert. Obgleich diese Rüst- und Reinigungszeiten bei der Bierherstellung einen verhältnismäßig geringen Anteil, gemessen am Gesamtgärprozess, der meist mehrere Wochen andauert, ausmachen, ist dies bei andersartigen Fermentationen nicht der Fall. So erfolgt die Vergärung von vielen Produkten (auch von Getränken) üblicherweise innerhalb einer sehr kurzen Zeit von weniger als 36 Stunden. Oftmals entfällt dabei der Prozessschritt der Reifung und Lagerung. In diesen Fällen werden dann entsprechend mehr Tanks 12 benötigt, da ständig ein hoher Anteil der zur Verfügung stehenden Tanks 12 befüllt, entleert und/oder gereinigt wird und deshalb nicht für die eigentliche Fermentation zur Verfügung steht. Dadurch erhöhen sich dementsprechend nicht nur die Investitionskosten, sondern auch die Anforderungen an die Befüll- und Entleerpumpen, sowie an die Reinigungsanlage und andere Apparaturen und Armaturen.

Bei anderen Gärungsprozessen wie bspw. bei der Bioethanolherstellung, der Milchsäureherstellung und bei der industriellen Essigproduktion werden deshalb kontinuierliche Verfahren bevorzugt bzw. angestrebt. Derzeit existieren hierfür verschiedene Verfahrensvarianten mit entsprechenden Anlagenkomponenten, die in aller Regel relativ komplex und damit auch kostenintensiv in der Anschaffung, im Betrieb wie auch in der Wartung und Instandsetzung sind. Weil viele dieser Schnellgärprodukte jedoch zunächst als sog. Rand- oder Nebenprodukte in bestehenden Betrieben entwickelt werden, verwendet man oftmals weiterhin die unwirtschaftlicheren, traditionellen Gärverfahren entsprechend Fig. 1. Ein Beispiel dafür liefert die Getränkeindustrie. Dort werden immer häufiger alternativ vergorene Produkte wie die sog. Bio-Limonaden oder das sog. Kwass, welche nur eine kurze Fermentation durchlaufen, mit der bestehenden Anlagentechnik hergestellt.

Eine Aufgabe der Erfindung ist, eine Vorrichtung zur Fermentation aus einer mehrstufigen Anordnung von zwei oder mehreren, miteinander gekoppelten Tanks zur Fermentation einer Flüssigkeit zur Herstellung eines Getränks zu schaffen, die die im Stand der Technik vorhandenen Nachteile reduziert oder vermeidet und die Wirtschaftlichkeit und ggf. die Ausbeute sowie die Qualität der mit der mehrstufigen Anordnung hergestellten Produkte verbessert.

Die obige Aufgabe wird durch eine Vorrichtung zur Fermentation gelöst, die aus einer mehrstufigen Anordnung von Tanks zur Fermentation besteht, die die Merkmale des Anspruchs 1 umfasst.

Eine weitere Aufgabe der Erfindung besteht darin, ein zumindest abschnittsweise kontinuierlich oder semikontinuierlich durchführbares Fermentationsverfahren zur Verfügung zu stellen.

Diese Aufgabe wird durch ein Verfahren zur kontinuierlichen oder semikontinuierlichen Fermentation von Flüssigkeiten zur Herstellung von Getränken innerhalb der erfindungsgemäßen Vorrichtung erreicht, das die Merkmale des Anspruchs 9 umfasst.

Merkmale vorteilhafter Weiterbildungen der mehrstufigen Anordnung der Vorrichtung zur Fermentation oder des Verfahrens zur kontinuierlichen oder semikontinuierlichen Fermentation ergeben sich aus den entsprechenden abhängigen Ansprüchen.

Ein hier beschriebener Tank zur Fermentation weist mindestens eine Befüllleitung zur vorzugsweise kontinuierlichen Zuführung von Flüssigkeit und mindestens eine Entnahmeleitung zur vorzugsweise kontinuierlichen Ableitung von vorteilhafterweise teilweise fermentierter Flüssigkeit auf, wobei eine der Leitungen einem sich im Tank zur Fermentation befindlichen Leitrohr zugeordnet ist. Der Tank zur Fermentation ist temperierbar. Des Weiteren sollte eine entsprechende Auslaufleitung zur Ableitung von sedimentierten Partikeln, Stoffen und/oder Organismen vorgesehen werden. Beschrieben ist ein sich innerhalb des Tanks zur Fermentation erstreckendes Leitrohr, dem die Entnahmeleitung zugeordnet sein kann. Das Leitrohr ist zur Unterseite hin offen. Das Leitrohr dient zur Lenkung und/oder Beruhigung der im jeweiligen Tank befindlichen Flüssigkeiten, da es eine definierte Strömung innerhalb des Tanks zur Fermentation bewirken kann. Zudem dient es bei Bedarf als Abscheidungsunterstützung von Partikeln, Stoffen und/oder Organismen.

Es kann das Leitrohr mit einer oder mehreren Heiz- bzw. Kühlzonen und/oder weiteren Ein- oder Anbauten ausgestattet sein. Vorzugsweise umfasst das Leitrohr einen zylindrischen oder leicht trichterförmigen Rohrabschnitt, der sich zur Entnahmeleitung hin verjüngt und zur Unterseite des Tanks zur Fermentation erweitert. Gemäß eines Beispiels sind die Befüllleitung sowie die Entnahmeleitung auf einem ähnlichen Niveau in einem oberen Bereich des Tanks angeordnet. Dabei ist die Befüllleitung wahlweise derart in Bezug auf die Tankinnenwand angeordnet, dass sich eine tangential gerichtete Strömung der Flüssigkeit im Tank ausbildet. Die Befüllleitung sollte möglichst unterhalb des angestrebten Füllstandniveaus münden. Wahlweise kann die Befüllung und/oder Entleerung jedoch auch über die Befüllleitung und/oder Entnahmeleitung in unterschiedlichen Höhenbereichen des Leitrohrs und/oder des Tanks erfolgen.

Typischerweise weist ein solcher Tank zur Fermentation in einem unteren Behälterbereich (Boden) eine Auslaufleitung auf. Diese ist zum einen wichtig, um die Organismen/Partikel kontinuierlich oder chargenweise abziehen zu können. Die Auslaufleitung ist zudem auch für die Reinigung notwendig, da es ansonsten schwierig ist, die Reinigungsflüssigkeit aus den Tanks abzuziehen, womit es auch schwierig wäre, den Tank vollständig zu entleeren. Die Auslaufleitung kann sich vorzugsweise an einen sich trichterförmig verjüngenden Bodenbereich (Konusspitze) anschließen.

Der Grundgedanke basiert darauf, dass eine oftmals bestehende Anlagentechnik (vgl. Fig. 1, Tanks 12) durch eine relativ einfache anlagen- und verfahrenstechnische Modifikation bei Bedarf ökonomischer genutzt werden kann. Dabei sollen vor allem die natürlichen Konvektionsströmungen, welche bei gegebenen Bedingungen während der Fermentation entstehen (vgl. Fig. 1, B, C und D), effizient genutzt werden, um vorzugsweise einen quasi-kontinuierlichen Prozess zu erreichen. Zudem sollen Fermentationsprodukte wie Gase und/oder Organismen sowie andere Stoffe dem Prozess bei Bedarf gezielt zugeführt und/oder abgeführt werden, um so den Prozess zu optimieren.

Ein Beispiel des Tanks zur Fermentation sieht vor, dass vorzugsweise im oberen Teil des Leitrohres Stömungs- und/oder Reinigungskörper wie z.B. Verteilschirme und/oder Spritzkugeln vorgesehen werden, die wahlweise mit der Entnahmeleitung für Gas und/oder Flüssigkeiten verbunden sind. Durch sie kann das Leitrohr bei einer Rückspülung gereinigt werden und im regulären Betrieb eine Strömungslenkung erfolgen. In diesem Zusammenhang können auch weitere Einbauten in oder an das Leitrohr erfolgen, die aufgrund ihrer Geometrie eine strömungsverbessernde Wirkung erzielen, die Reinigung erleichtern und/oder die Partikelsedimentation fördern. Hinzu kommt, dass auch die Gaszufuhr und/oder Gasabfuhr aus dem Tank ermöglicht sein soll. Dabei muss jedoch weiterhin eine gründliche Reinigung aller Teile und Teilbereiche des Tanks zur Fermentation erfolgen können.

Vorzugsweise weist der Tank zur Fermentation eine abschnittsweise zylindrische Form mit einer vertikalen Mittelachse auf. Zudem sollte der Tank mit einem Kühlmantel, der aus mehreren Kühlzonen besteht, versehen sein. Ebenso ist eine Raumkühlung denkbar, so dass durch eine geeignete Temperierung des Tanks die natürliche Strömung innerhalb des Tanks beeinflusst werden kann (vgl. Fig. 1B, C; D). Wahlweise kann jedoch auch das Leitrohr (ggf. zusätzlich oder ausschließlich) temperiert werden, wobei der Tank dann idealerweise isoliert sein sollte. Die Einstellung eines Temperaturgradienten innerhalb der Flüssigkeit im Tank erfolgt dann vorzugsweise ausschließlich mittels des Leitrohrs. Des Weiteren sollte am tiefsten Punkt des Tankbodens, oder vorzugsweise an der unteren Konusspitze des Tanks, eine Auslaufleitung installiert sein, um einen Teil des Tankinhalts bei Bedarf kontinuierlich oder chargenweise von unten abziehen zu können. Der Kernstrom der Würze wird dagegen in einer bevorzugten Verfahrensweise durch das im Tank befindliche Leitrohr nach oben hin abgezogen und wahlweise in den nächsten Tank geleitet. Des Weiteren sollte in einer bevorzugten Ausführung des Tanks zur Fermentation die Installation für Gas- bzw. Fluidanschlüsse im unteren Teil des Tanks vorgesehen werden. Durch sie soll bei Bedarf eine zusätzliche kontinuierliche oder chargenweise Eindosierung bestimmter, vorzugsweise während der Fermentation produzierter Stoffe erfolgen können, die sich positiv auf den Prozess auswirken. So kann bspw. die Einleitung von Gas ein erneutes Aufwirbeln von sedimentierten Teilchen, Stoffen und/oder Organismen bewirken und Organismen (z.B. Aerobier und/oder Anaerobier) gezielt in ihrem Wachstum und in ihrem Stoffwechsel beeinflusst werden. Außerdem kann dadurch das Aromaprofil sowie die chemisch-physikalische Zusammensetzung des Fermentates und auch die Strömung gezielt gesteuert werden. Um entstehende oder zugeführte Gase abzuleiten, können zusätzlich entsprechende Einrichtungen im oder am Leitrohr und/oder im oder am Tank vorgesehen werden. In diesem Zusammenhang gilt es auch die Schaumbildung der jeweiligen Produkte zu berücksichtigen. So kann es bspw. sinnvoll sein, eine Querschnittserweiterung des Leitrohres vorzusehen und/oder entsprechende Steigräume im Tank und/oder im Leitrohr zu gewährleisten. In einer weiteren Ausführungsform wird die Installation von zusätzlichen Temperiereinrichtungen bzw. Kühlzonen vorgesehen. Diese können nicht nur in oder an dem Leitrohr selbst, sondern auch unterhalb des Leitrohrs und/oder des Mantelbereichs angebracht werden. Sie sollen die natürlichen Konvektionsströmungen unterstützen, indem sie die Dichte des Mediums lokal verändern.

Zweckmäßigerweise ist das im Tank angeordnete Leitrohr (Leitungsabschnitt) annähernd koaxial zum Tank angeordnet. Dabei kann das Leitrohr einen Durchmesser aufweisen, der wenigstens 15% und nicht mehr als 70% des Durchmessers des Tanks selbst entspricht. Als vorteilhaft hat sich ein Durchmesser des bevorzugt abschnittsweise zylindrischen Leitrohrs herausgestellt, der ungefähr 20 bis 50% des Durchmessers des Tanks zur Fermentation entspricht.

Ein Beispiel des Tanks zur Fermentation sieht vor, dass das Leitrohr über seine Länge konisch oder unregelmäßig geformt sein kann. Es kann grundsätzlich jede zweckmäßige Gestalt aufweisen, die geeignet ist, die Strömung, die Gaszu- und/oder -ableitung, sowie die Partikelzuführung- und/oder -sedimentation in der gewünschten Weise zu lenken und zu beeinflussen. Wahlweise kann das Leitrohr daher zusätzlich temperierbar und/oder thermisch isoliert sein. Weiterhin kann das Leitrohr an seiner Außenseite strömungslenkende und/oder -leitende Leiteinrichtungen, insbesondere Leitbleche o. dgl. aufweisen, die wahlweise abschnittsweise oder über die gesamte Länge des Leitrohrs angeordnet sein können. Derartige strömungslenkende und/oder -leitende Leiteinrichtungen wie Leitbleche o. dgl. können wahlweise auch an der Innenseite des Tanks angeordnet sein.

Weiterhin kann der Tank zur Fermentation Einbauten für die Einleitung von Gasen und/oder Fluiden, für die Reinigung sowie ggf. weitere Temperiervorrichtungen aufweisen, die wahlweise im unteren, im zentralen und/oder im äußeren und/oder oberen Tankbereich angeordnet sein können. Darüber hinaus kann der Tank ggf. auch durch Kühlzonen oder Raumtemperierung erhitzt/gekühlt werden. Schließlich können Pumpen, Ventilknoten, Mess-, Regel- und Dosiervorrichtungen, z. B. zwischen der Befüllleitung und der Entnahmeleitung, oder an sich bekannte Armaturen wie bspw. Gasableitungen vorgesehen sein.

Ein Beispiel des Tanks zur Fermentation weist einen sich trichterförmig verjüngenden und in eine Entnahmeleitung oder Befüllleitung mündenden, geschlossenen, oberen Bereich des Leitrohrs auf. Der obere Bereich des Leitrohrs kann wahlweise immer zusätzlich oder ausschließlich zu den im Tank installierten Gaszu- und/oder -ableitungen mit einer weiteren Gaszu- oder -ableitung versehen sein.

Die Erfindung umfasst eine Vorrichtung zur Fermentation, die aus einer mehrstufigen Anordnung von zwei oder mehreren, miteinander in einer Kaskade gekoppelten Tanks zur Fermentation gemäß einer oder mehrerer verschiedener der zuvor beschriebenen Ausführungsvarianten besteht. Bei der Vorrichtung ist eine Entnahmeleitung eines ersten Tanks zur Fermentation mit einer Befüllleitung eines zweiten Tanks zur Fermentation verbunden, dessen Entnahmeleitung ggf. zu einer weiteren Befüllleitung eines dritten Tanks zur Fermentation führt etc. Die Entnahmeleitung eines vorgeordneten Tanks ist also mit der Befüllleitung eines unmittelbar nachgeordneten Tanks verbunden. Da sich jedoch, wie in Fig. 1, Abb. D gezeigt, die natürliche Konvektion bei kalten Temperaturen dichteabhängig umdrehen kann, sollte die Möglichkeit vorgesehen werden, die Befüllrichtung und Entnahmerichtung zumindest einzelner Tanks entsprechend zu verändern. Dies bezieht sich auch auf einen kaskadierten Betrieb der mehrstufigen Anordnung. In diesem Fall wird die Flüssigkeit mittig über das Leitrohr derart eingeführt, dass sich wahlweise z.B. eine pfropfenförmige oder eine tangentiale Strömung in Bezug auf eine Tankinnenwand ausbildet. Die Zufuhr der Flüssigkeit erfolgt vorzugsweise im oberen, äußeren Mantelbereich des Tanks über eine Befüllleitung. Weiterhin kann vorgesehen sein, dass die zusätzliche Auslaufleitung im Bodenbereich mit zumindest einer der oberen Befüllleitungen verbunden ist, um so bspw. sedimentierte Organismen dem System erneut gezielt zuzuführen. Auch eine Gasableitung zumindest eines Tanks sollte sinnvollerweise bei zumindest einem gleichen oder anderen Tank mit einer Gaszuleitung in den Bodenbereichen des Tanks verbunden werden, so dass - ggf. mit Hilfe einer zusätzlichen Förderpumpe - das bei der Gärung gewonnene Gas an bestimmten Stellen dem Prozess zumindest teilweise erneut zugeführt werden kann.

Durch die äußere Tankkühlung fällt die Würze normalerweise im Mantelbereich der Tanks ab. Aufgrund der verringerten Strömungsgeschwindigkeit an der Wandung der Tanks sedimentieren zudem verstärkt Partikel, die sich im unteren Bereich des Tanks sammeln und von dort kontinuierlich oder chargenweise abgezogen werden können. Die wahlweise tangentiale Strömung der eingeleiteten Flüssigkeit sowie der Strömungswiderstand, der durch das Leitrohr entsteht, sowie ggf. weitere Einbauten in und an dem Leitrohr können diesen Sedimentationseffekt zusätzlich verstärken. Der Kernstrom (Aufwärtsströmung) der Würze wird dagegen normalerweise durch das im Tank befindliche Leitrohr nach oben hin abgezogen und in den nächsten Tank geleitet. Durch das Einleiten und/oder Ausleiten von Gasen und/oder anderer Partikel und/oder Fluiden in das oder aus dem Leitrohr oder in den oder aus dem äußeren Mantelbereich kann die Strömungsrichtung und die Strömungsgeschwindigkeit zusätzlich gesteuert werden. Zudem können dadurch das Aromaprofil sowie die chemisch-physikalische Zusammensetzung des Produktes und weitere Fermentationsprozesse gezielt beeinflusst werden. Durch mehrere erfindungsgemäß hintereinander angeordnete Stufen (mehrstufige Anordnung) kann das Produkt bei Bedarf z.B. von Tank zu Tank immer weiter abgekühlt und können somit zudem immer mehr kleinere Partikel abgeschieden werden. Die abgeschiedenen und abgezogenen Teilchen und/oder Organismen können dann wahlweise entfernt und anderweitig verwendet bzw. verworfen und/oder zumindest teilweise durch Zudosierung dem Prozess an geeigneter Stelle wieder zugeführt werden. Das fertige, vorgeklärte Produkt wird aus dem letzten erfindungsgemäßen Tank vorzugsweise von oben abgezogen. Bei Bedarf können herkömmliche Pufferbehälter den erfindungsgemäßen Tanks zur Fermentation oder der mehrstufigen Anordnung zur Fermentation vor- und/oder nachgeschaltet werden, um einen kontinuierlichen Zu- und Abfluss in das bzw. aus dem System zu gewährleisten.

Der kontinuierliche Produktstrom kann bspw. über eine Druckregulation unterstützt bzw. gesteuert werden. Eine kontinuierliche Verarbeitung und Förderung von den zu fermentierenden Flüssigkeiten kann besonders dann vereinfacht erfolgen, wenn der Druck im vorgeschalteten Tank größer ist als im nachgeschalteten Tank bzw. in den nachgeschalteten Tanks.

Zur Unterstützung der Produktstromförderung kann eine Förderpumpe in die Entnahmeleitung des letzten Tanks eingebracht werden, so dass ein verhältnismäßig ansteigender Unterdruck der nachgeschalteten Tanks im Vergleich zu den vorgeschalteten Tanks entsteht. Dadurch erfolgt jedoch der Flüssigkeitseintrag in die Tanks über dem jeweiligen Füllstandniveau, was je nach Produkt zu einer verstärkten Schaumbildung innerhalb des Tanks führen kann. Um dies zu verhindern, kann jeweils eine Förderpumpe in die Verbindungsleitung zweier aufeinander folgender Tanks eingebaut werden, so dass die Einbringung von Substrat aus dem vorgeschalteten Tank zur Fermentation jeweils unterhalb des Flüssigkeitsspiegels erfolgt. Zudem können auch Zusätze eingesetzt werden, die eine Schaumbildung verringern. Um wahlweise diverse Zusätze wie Organismen, Säuren, Chemikalien, Aromen, Gase, Würze, Stabilisatoren, andere Partikel usw. in die jeweiligen Entnahmeleitungen bzw. Verbindungsleitungen einzudosieren, können zusätzlich entsprechende Einrichtungen wie Dosageventile, Dosagepumpen, Entgasungsvorrichtungen usw. integriert werden. Prinzipiell erscheint daher die Installation eines entsprechenden Ventilknotens für die Verbindungs- und Entnahmeleitungen sinnvoll. Dadurch können zudem ggf. einzelne Tanks bei Bedarf vom System abgekoppelt oder integriert werden, so dass bspw. eine Reinigung der einzelnen Behälter erfolgen kann und/oder Produktionsspitzen kompensiert werden können, indem die Kapazitäten bzw. die Durchflussraten beeinflusst werden. Zudem kann dadurch eine eventuelle Dosage oder eine gezielte Produktstromförderung vereinfacht werden. Des Weiteren können Temperierungsvorrichtungen auch zwischen den Entleer- und Befülleitungen kaskadierter Tanks angebracht werden. Durch sie können beispielsweise biochemische Prozesse katalysiert, bzw. Ausfällungsreaktionen und ähnliches beschleunigt werden.

Bei einer weiteren Ausführungsvariante der Vorrichtung ist zumindest eine der Gasableitungen mit der Gaszuleitung des/der vor- und/oder nachgeschalteten Tanks verbunden. Wahlweise können die Tanks der mehrstufigen Anordnung jeweils unterschiedlich und/oder individuell temperiert werden. Durch je nach Bedarf gleich oder unterschiedlich starke Abkühlung oder Erwärmung bzw. Temperierung kann die Fermentation in optimierter Weise beeinflusst und gesteuert werden, indem gezielt Kühlzonen und/oder Zonen erwärmter Flüssigkeit gebildet werden. Die Fermentation kann weiterhin durch Vorrichtungen zum Einleiten von Gas und/oder Fluid (z. B. Luft, Organismen, Stabilisatoren, etc.) optimiert werden.

Neben den genannten Vorteilen bietet eine derartige mehrstufige Anordnung weitere Vorzüge. So kann bspw. eine Veränderung der entstehenden Gasreinheit in den verschiedenen Tanks dazu genutzt werden, um eine ökonomische Gasaufbereitung, wie eine Kohlendioxid-Rückgewinnung, zu gestalten. Daher sollten vorzugsweise alle Gasentnahme- und Gasdosageleitungen entsprechend miteinander verbunden werden, um so eine gezielte Steuerung zu ermöglichen. Auch die unterschiedliche Partikelgrößenverteilung, welche bei entsprechender Betriebsweise von Kaskaden resultiert, kann verfahrenstechnisch genutzt werden. So könnte bspw. die Kalttrubentfernung sowie die Organismenabscheidung besser gesteuert werden. Außerdem könnte sich die Reinheit der abgezogenen Stoffe, Partikel und/oder Organismen erhöhen, was eine Aufbereitung oder Verwerfung bestimmter Inhaltsstoffe ökonomischer machen würde.

Das weitere Ziel der Erfindung wird mit einem Verfahren zur kontinuierlichen oder semikontinuierlichen Fermentation von Flüssigkeiten mit den Merkmalen des unabhängigen Verfahrensanspruchs erreicht. So betrifft die Erfindung ein Verfahren zur kontinuierlichen oder semikontinuierlichen Fermentation von Flüssigkeiten zur Herstellung von Getränken innerhalb der erfindungsgemäßen Vorrichtung mit den folgenden Schritten:• dass in mindestens einem der Tanks die zu fermentierende Flüssigkeit über mindestens eine Befüllleitung in einen oberen Bereich des mindestens einen der Tanks zur Fermentation eingeleitet wird,• dass mittels eines sich innerhalb des mindestens einen der Tanks erstreckenden Leitrohrs die Flüssigkeit gelenkt und/oder beruhigt wird,• dass die Flüssigkeit zu mindestens einer Entnahmeleitung geleitet wird, in die das Leitrohr mündet; und dass sich in der Flüssigkeit enthaltene Partikel und/oder Organismen in einem unteren, sich trichterförmig verjüngenden Bereich des mindestens einen der Tanks absetzen und kontinuierlich und/oder zyklisch über eine Auslaufleitung (50), die nicht mit dem Leitrohr (36) in Verbindung steht, aus einem der Tanks abgezogen werden.

Die Flüssigkeit kann durch zwei oder mehrere, in einer mehrstufigen Anordnung angeordnete Tanks zur Fermentation kontinuierlich oder semikontinuierlich geleitet werden, die derart miteinander verbunden sind, dass die Entnahmeleitung des ersten Tanks mit der Befüllleitung des zweiten Tanks verbunden ist, und dass die Entnahmeleitung des zweiten Tanks mit der Befüllleitung des drittenTanks verbunden ist, usw. Es können jedoch auch verschiedene Tanks wahlweise unterschiedlicher Bauform, zur Fermentation so miteinander verbunden bzw. geschaltet werden, dass die Befüllung und Entleerung von zumindest einem Tank in umgekehrter Richtung erfolgt, so dass die Befüllleitung von oben über das Leitrohr mündet und die Entnahmeleitung mit dem oberen Bereich des Tanks verbunden ist. Zudem können erfindungsgemäßen Tanks bekannte Vorrichtungen vor- und/oder nachgeschaltet sein, welche die Fermentation auf positive Weise beeinflussen sollen.

Es erfolgt die kontinuierliche Befüllung der Tanks jedoch jeweils in ihrem oberen Bereich, wahlweise tangential zu der Innenwand des Tanks. Eine andersartige Befüllung und Entleerung zusätzlich oder ausschließlich in anderen Bereichen des Tanks soll dadurch jedoch nicht ausgeschlossen sein. Durch die äußere und/oder innere Kühlung des Tanks kann sich die Flüssigkeit entlang der sich vertikal erstreckenden Längsrichtung des länglichen Tanks und/oder des länglichen Leitrohrs entsprechend abkühlen und/oder erwärmen und dabei nach unten sinken oder nach oben aufsteigen. Alternativ hierzu kann sich die Flüssigkeit entlang der sich vertikal erstreckenden Längsrichtung des länglichen Tanks von unten nach oben abkühlen, wobei sie gleichzeitig nach oben steigt.

Dabei können sich in der Flüssigkeit enthaltene Partikel und/oder Organismen im unteren Bereich des Tanks absetzen und kontinuierlich und/oder zyklisch abgezogen werden.

In diesem Zusammenhang kann es von Vorteil sein, wenn zumindest ein Teil der vom unteren Bereich des Tanks abgezogenen Partikel und/oder Organismen eines Tanks einem oder mehreren der vor oder nach dem jeweiligen Tank angeordneten Tanks wieder zugeführt wird.

Die eingeleiteten Gase und/oder die entstehenden Fermentationsgase hingegen steigen tendenziell im Leitrohr bzw. im Tank auf, so dass entsprechende Einbauten (Ableitelemente) vorgesehen werden sollten. Auch hier kann eine erneute Zuführung von Gasfraktionen an anderen Stellen innerhalb des Prozesses oder an anderen Stellen der Tanks zur Fermentation erfolgen.

Normalerweise erfolgt die Tankkühlung bei der Hauptgärung und der Reifung über Kühlzonen, welche von außen an der Wandung des Tanks angebracht sind. Die einzelnen Kühlzonen können gezielt gesteuert werden, so dass sich Temperaturgradienten innerhalb des Tanks einstellen. Durch die Kühlung fällt im Tankinneren die Flüssigkeit tendenziell an der Innenwandung des Tanks ab und es bildet sich eine vertikal nach unten gerichtete Abwärtsströmung (Randströmung) aus. Der innere Teil des Tanks weist hingegen höhere Temperaturen auf, so dass zur Mittelachse des Tanks hin eine vertikal gerichtete Aufwärtsströmung entsteht (vgl. Fig. 1, Abb. B und C), die nun durch das erfindungsgemäß installierte Leitrohr von unten nach oben geführt wird. Um eine derartige Strömung auszubilden oder zu unterstützen, kann zudem (oder ausschließlich) auch das Leitrohr wahlweise über einzelne oder verschiedene, separat angesteuerte Kühl-/Heizzonen temperiert werden, wobei wahlweise die nach innen oder außen gerichtete Seite des Leitrohrs teilweise oder vollständig isoliert werden kann. Außerdem kann das natürliche Strömungsverhalten gezielt verändert werden, indem erfindungsgemäß zusätzlich Gas und/oder ein anderes Fluid bzw. Partikel mit verhältnismäßigem Auftrieb oder Abtrieb in das Leitrohr oder in den Randbereich von oben oder unten in den Tank eingeleitet werden. Die Strömung und die ablaufenden biochemischen Reaktionen können zusätzlich durch weitere temperierbare Körper, welche sich vorteilhafterweise zentral unterhalb des Leitrohrs und/oder am unteren, äußeren Mantelbereich befinden, beeinflusst werden. Da sich bei der Kaltlagerung/Kaltreifung die natürliche Strömungsrichtung umdrehen kann, sollte die Möglichkeit vorgesehen werden, das System ggf. in umgekehrter Richtung zu betreiben. In diesem Fall erfolgt die vorzugsweise kontinuierliche Befüllung, wahlweise auch mittels einer tangentialen Strömung, über das mittig installierte und ggf. nach Bedarf temperierbare Leitrohr. Dort fällt die dichter werdende Flüssigkeit zunehmend ab und evtl. enthaltene Partikel, Organismen und/oder andere Stoffe sammeln sich am Boden des Tanks. Zeitgleich kann entstehendes Fermentationsgas quasi im Gegenstrom aufsteigen, welches dann im oberen Teil des Leitrohrs bzw. Mantelbereich des Tanks wahlweise abgeführt werden kann. Am Randbereich steigt die Flüssigkeit erneut auf und wird vom oberen Bereich des Tanks abgeführt. Auch hier können eine Gas-, Stoff- und/oder Partikeleinleitung mit entsprechendem Auf- bzw. Abtrieb sowie ggf. eine Installation von Einbauten im Tank in oder an das Leitrohr sowie entsprechende Temperiervorrichtungen Vorteile haben.

Aufeinander folgende Tanks können einen zu- oder abnehmenden Innendruck im Tank aufweisen. Zudem kann die Flüssigkeit in aufeinander folgenden Tanks zunehmend abgekühlt oder auch erwärmt und aus ihr zunehmend mehr und/oder kleinere Partikel und/oder Organismen abgeschieden werden.

Eine Variante des erfindungsgemäßen Verfahrens kann vorsehen, dass die Flüssigkeit in aufeinander folgenden Tanks zunehmend erwärmt oder gekühlt wird, um das Wachstum bestimmter Organismen zu beeinflussen. Wahlweise oder zusätzlich kann die Flüssigkeit in aufeinander folgenden Tanks auch mit bestimmten Gasen beaufschlagt werden, um auf diese Weise das Wachstum bestimmter Organismen zu beeinflussen. In diesem Zusammenhang ist die Zu- und/oder Abführung weiterer Stoffe, die beispielsweise den pH-Wert, den Druck, die Temperatur und/oder die Substratzusammensetzung beeinflussen, möglich. Eine Ausführungsvariante des erfindungsgemäßen Verfahrens kann daher vorsehen, dass in wenigstens eine Befüllleitung eines der Behälter wenigstens ein Zusatzstoff eindosiert wird. Zudem kann in wenigstens einen Gasanschluss im unteren und/oder oberen Behälterbereich Fermentationsgas und/oder andere Gase eingeleitet werden.

Mit der erfindungsgemäßen Vorrichtung zur Fermentation sowie mit dem erfindungsgemäßen Verfahren werden Getränke hergestellt. Wie auch hier beschrieben, eignen sich die Vorrichtung und das Verfahren zur Erzeugung von Bioethanol oder zur Produktion von Methangas. Wie hier beschrieben, kann es auch zur Verarbeitung von Molke und/oder zur Herstellung von Essigsäure oder Milchsäure sowie zur Partikelauffächerung und Partikelabtrennung dienen.

Die Erfindung wird nachfolgend anhand eines bevorzugten Ausführungsbeispiels unter Bezugnahme auf die beiliegenden Zeichnungen näher erläutert. Gleiche Elemente oder gleich wirkende Elemente in den Zeichnungen sind hierbei mit gleichen Bezugszeichen versehen und werden deshalb teilweise nicht mehrfach erläutert. Grundsätzlich soll das anliegende Ausführungsbeispiel die Erfindung illustrieren, ist jedoch keinesfalls einschränkend zu verstehen.
Fig. 1 zeigt in den Darstellungen A-F vereinfacht ein aus dem Stand der Technik bekanntes chargenweises Gärverfahren in einem modernen zylindrokonischen Tank zur Fermentation von Bier. Im Schritt A wird der Tank von unten befüllt, das Substrat zumindest teilweise während der Schritte B-D fermentiert und der Tank anschließend wieder von unten im Schritt E, entleert, wonach eine Tankreinigung im Schitt 1F erfolgt.
Fig. 2A zeigt vereinfacht eine Gegenüberstellung eines herkömmlichen Tanks zur Fermentation und eines vereinfacht dargestellten, modifizierten Tanks zur Fermentation.
Fig. 2B zeigt vereinfacht die Strömungs- und Prozessabläufe während einer beispielhaften Hauptgärung in einem herkömmlichen Tank zur Fermentation (links), verglichen mit den Grundabläufen in einem vereinfacht dargestellten, modifizierten Tank zur Fermentation, welcher in bevorzugter Richtung betrieben wird (rechts).
Fig. 2C zeigt vereinfacht die Strömungs- und Prozessabläufe während einer beispielhaften Kaltreifung/Kaltlagerung in einem herkömmlichen Tank zur Fermentation (links), verglichen mit den Grundabläufen in einem vereinfacht dargestellten, modifizierten Tank zur Fermentation (rechts), der hier in umgekehrter Richtung betrieben wird.
Fig. 2D zeigt eine weitere vereinfachte Ausführungsform der modifizierten Tanks (rechts) in Gegenüberstellung mit einem herkömmlichen Tank zur Fermentation (links).
Fig. 2E zeigt eine vereinfachte Ausführungsform des modifizierten Tanks, bei dem eine Gasableitung im oberen Bereich des Leitrohrs installiert ist und die ansonsten weitgehend der Darstellung aus Fig. 2D ähnelt.
Fig. 2F zeigt eine vereinfachte Ausführungsform des modifizierten Tanks, bei dem eine Gasableitung im oberen Bereich des Leitrohrs installiert und die in Vergleich zu Fig. 2E in um- gekehrter Richtung betrieben wird.
Fig. 3 zeigt vereinfacht eine bevorzugte Bauart des modifizierten Tanks zur Fermentation.
Fig. 4 illustriert einige mögliche Modifikationen der einzelnen Bestandteile und/oder mögliche Ausführungsbeispiele des Tanks bzw. Leitrohrs.
Fig. 5 zeigt vereinfacht eine beispielhafte mehrstufige Anordnung mit fünf nacheinander angeordneten Tanks zur Erzielung eines kontinuierlichen Fermentationsverfahrens.

Für gleiche oder gleich wirkende Elemente werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dar, wie der Tank zur Fermentation, die erfindungsgemäße Vorrichtung und das erfindungsgemäße Verfahren ausgestaltet sein können.

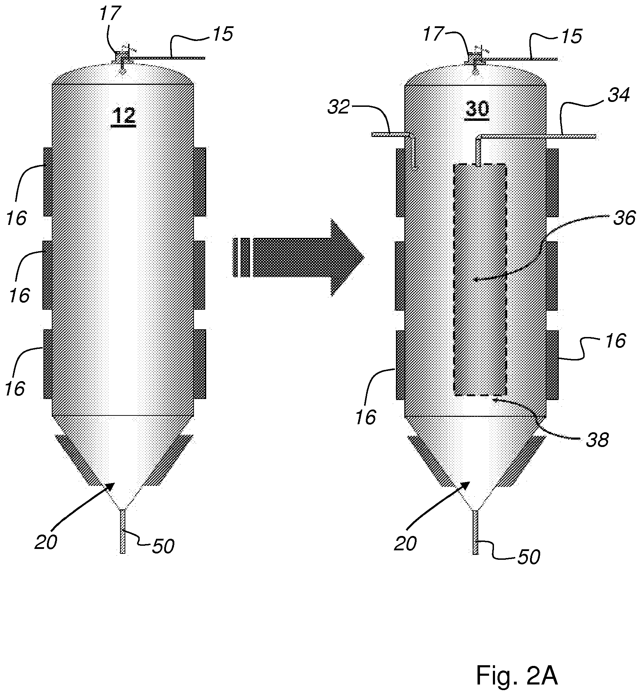

Die schematische Schnittdarstellung der Fig. 2A zeigt vereinfacht eine Gegenüberstellung eines herkömmlichen Tanks 12 zur Fermentation (links) und eines modifizierten Tanks 30 zur Fermentation (rechts). Der Tank 30 zur Fermentation weist mindestens eine Befüllleitung 32 zur vorzugsweise kontinuierlichen Zuführung einer frischen Flüssigkeit und mindestens eine Entnahmeleitung 34 zur ebenfalls bevorzugt kontinuierlichen Ableitung einer vorzugsweise zumindest teilweise fermentierten Flüssigkeit auf. Innerhalb des Tanks 30 zur Fermentation ist ein vertikales Leitrohr 36 angeordnet, welches mit der Entnahmeleitung 34 bzw. mit der Befüllleitung 32 verbunden ist. Obwohl in den nachfolgenden Zeichnungen das Leitrohr 36 in der Mitte des Tanks 30 angeordnet ist, soll dies nicht als eine Beschränkung aufgefasst werden. Es ist für einen Fachmann selbstverständlich, dass das Leitrohr 36 nicht ausschließlich um die Mittelachse 11 des Tanks 30 angeordnet sein muss. Das Leitrohr 36, welches zur Beruhigung und/oder Lenkung der Strömung sowie als Sedimentationshilfe dient, ist zu seiner Unterseite 38 hin offen. Um die sedimentierten Partikel zu entfernen, sowie die Flüssigkeit nach der Reinigung abzuführen, befindet sich an der tiefsten Stelle des Tanks 30 (hier die untere Konussspitze 20) eine Auslaufleitung 50. Über sie kann während des Prozesses ein Teil des Inhalts des Tanks 30 bei Bedarf kontinuierlich oder chargenweise abgeführt werden. Da eine nachträgliche Installation der Einbauten in herkömmliche Tanks 12 erfolgen kann, ist der Tank 30 vorzugsweise mit den gleichen Armaturen und Vorrichtungen wie z.B. Domarmatur 17 mit Reinigungsanschluss 15, Gasanschluss, Vakuumventil sowie Probenahmevorrichtungen, Temperatur- und Drucksonden, Füllstandsmessern, Kühlzonen 16 usw. wie herkömmliche Tanks 12 zur Fermentation (vgl. auch Fig. 1) ausgestattet. Ebenso kann das Leitrohr 36 mit entsprechenden Elementen bestückt sein. Zudem sind zylindrokonische Tanks 30 solchen mit geraden Böden oder Klöpperböden gegenüber zu bevorzugen.

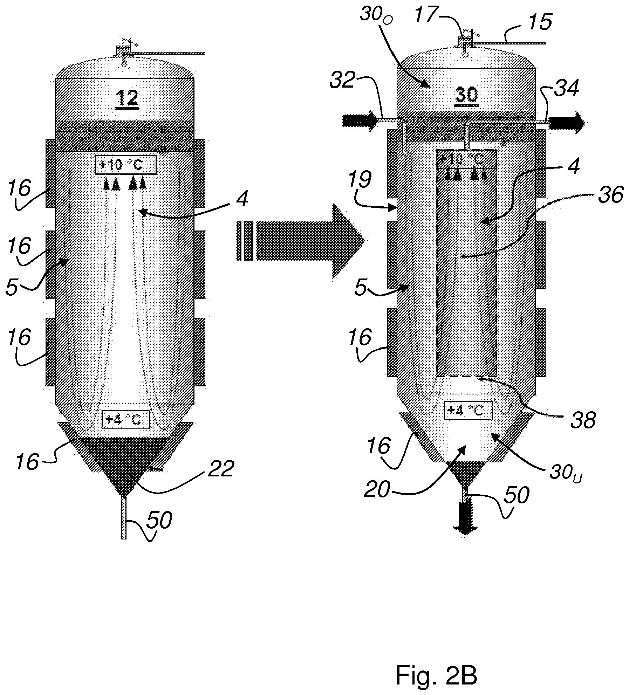

Die vereinfachte Prozessdarstellung in Fig. 2B zeigt vereinfacht eine Gegenüberstellung eines herkömmlichen Prozessverlaufes während einer beispielhaften Hauptgärung in einem herkömmlichen Tank 12 zur Fermentation (links) im Vergleich zu dem gleichen Prozessstadium in einem modifizierten Tank 30 zur Fermentation (rechts). Im herkömmlichen Tank 12 zur Fermentation wird der Tankinhalt üblicherweise durch die von außen angebrachten Kühlzonen 16, oder durch eine Raumkühlung abgekühlt. Dadurch nimmt die Dichte der Flüssigkeit, welche sich in der Nähe der Wandung 19 befindet, zu. Es kommt dementsprechend zu einer vertikal gerichteten Abwärtsströmung 5 in einem äußeren, wandnahen Tankbereich. Diese wiederum, und die höheren Temperaturen in der Tankmitte, führen zu einer vertikal gerichteten Aufwärtsströmung 4 (Kernströmung) im Tankinneren, in Nähe der Mittelachse 11 (siehe Fig. 1). Zudem sinken während des Prozesses üblicherweise zunehmend mehr Partikel 22 in die Konussspitze 20 des Tanks 12 ab. Diese können über die Auslaufleitung 50 vorsichtig abgezogen und weiterverarbeitet und/oder verworfen werden. Das dargestellte Beispiel macht sich mit Hilfe des modifizierten Tanks 30 diese natürlichen Strömungs- und Sedimentationsvorgänge zu Nutze. So soll durch das mittig installierte Leitrohr 36 die in diesem Fall aufwärts gerichtete Aufwärtsströmung 4 (Kernströmung) kontinuierlich mittels der Entnahmeleitung 34 abgeführt werden können. Auch die abgeschiedenen Partikel 22 können kontinuierlich oder chargenweise aus dem unteren Tankbereich 30_{U} über die Auslaufleitung 50 entfernt und wahlweise weiterverarbeitet und/oder verworfen werden. Um das System im Gleichgewicht zu halten, sollte das abgeführte Volumen durch neue Flüssigkeit ausgeglichen werden. Diese wird hier über die im oberen Tankbereich 30_{O} angebrachte Befüllleitung 32 zugeführt.

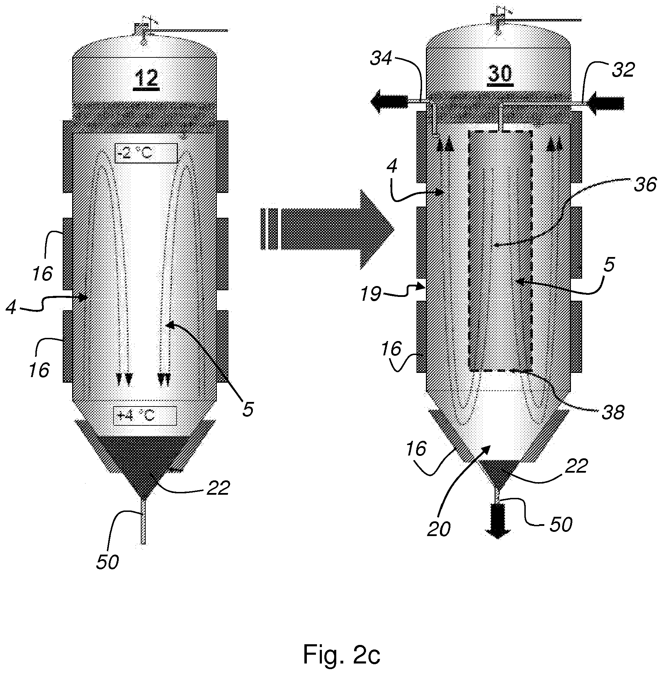

Die vereinfachte Prozessdarstellung in Fig. 2C zeigt vereinfacht eine Gegenüberstellung eines herkömmlichen Prozessverlaufes während einer beispielhaften Kaltreifung / Kaltlagerung in einem herkömmlichen Tank 12 zur Fermentation (links), im Vergleich zu dem gleichen Prozessstadium in einem erfindungsgemäß modifizierten Tank 30 zur Fermentation (rechts). Im herkömmlichen Tank 12 zur Fermentation wird der Tankinhalt bei diesem Prozessschritt meistens stark mit Hilfe der Kühlzonen 16 oder wahlweise über eine Raumkühlung abgekühlt. Durch eine Dichteanomalie der Flüssigkeit kann es dabei zu einer Strömungsumkehrung innerhalb des herkömmlichen Tanks 12 kommen. Demnach steigt die kältere, aber weniger dichte Flüssigkeit im äußeren Tankbereich vertikal nach oben und erzeugt eine Aufwärtsströmung 4. Diese Aufwärtsströmung 4, sowie die höheren Temperaturen im Tankinneren, bewirken eine vertikal abwärts gerichtete Abwärtsströmung 5 (Kernströmung). Auch hier sedimentieren die noch enthaltenen Partikel 22 in die Konussspitze 20. Durch die Modifikation des Tanks 30 kann auch diese Strömungsumkehrung problemlos genutzt werden. Eine Möglichkeit dazu illustriert die rechte Abbildung der Fig. 2C. Die Befüllleitung 32 ist mittig über dem Leitrohr 36 installiert, so dass die Strömung ebenfalls zentral nach unten geleitet wird und eine Abwärtsströmung 5 erzeugt. In den Außenbereichen des Tanks 30 nimmt die Flüssigkeitsdichte durch die weitere Abkühlung unterhalb von z.B. 4 °C ab und so steigt die Flüssigkeit im Bereich der Wandung 19 nach oben (Aufwärtsströmung 4), von wo aus sie entsprechend über eine Entnahmeleitung 34 abgeführt werden kann. Die Entnahme kann dabei vorzugsweise kontinuierlich erfolgen. Auch hier können Partikel 22 sedimentieren, welche sich in der Konussspitze 20 anreichern, so dass sie von dort, wahlweise kontinuierlich oder chargenweise, über die Auslaufleitung 50 abgezogen werden können.

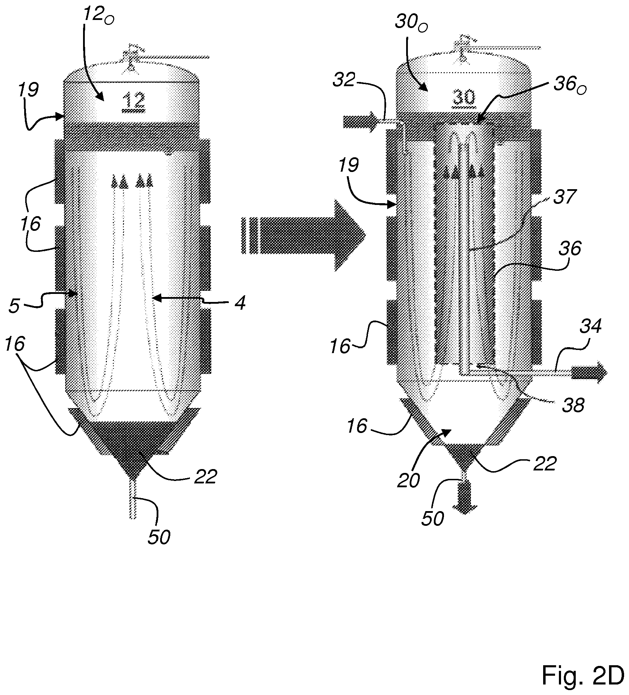

Die nachfolgend beschriebenen Figuren 2D-F illustrieren, dass das Verfahren, welches ein Leitrohr 36 in einem Tank 30 vorsieht, auf unterschiedliche Art und Weise modifiziert werden kann. So können beispielsweise zusätzliche Rohrleitungen wie z.B. 37 vorgesehen werden, durch die eine Flüssigkeitszu- und/oder -abführung erfolgen kann. Derartige Rohre 37 können, wie hier gezeigt von unten, oder auch von oben oder seitlich in das Leitrohr eingebaut werden. Des Weiteren können zusätzlich Gase, vorzugsweise im oberen Leitrohrbereich 36_{O}, mittels entsprechenden Gasableitungen 60 ab- und/oder zugeführt werden. Um eine bessere Reinigung des Systems zu gewährleisten, sollten die Gas- und/oder Flüssigkeitsein- und -ausleitvorgänge (z.B. über Leitungen 37 und/oder 60) idealerweise über offene Reinigungskörper wie Spritzkugeln o. ä. erfolgen. Diese werden hier jedoch zur Vereinfachung nicht dargestellt.

Fig. 2D zeigt daher vereinfacht eine Gegenüberstellung eines herkömmlichen Tanks 12 (links) im Vergleich zu dem modifizierten Tank 30 zur Fermentation (rechts) gemäß einer weiteren Ausführungsform der Erfindung. Der herkömmliche Tank 12 und der erfindungsgemäß modifizierte Tank 30 besitzen hier beispielsweise von außen angebrachte Kühlzonen 16. Dadurch kann die Dichte der Flüssigkeit, welche sich in der Nähe der Wandung 19 befindet, gezielt beeinflusst werden. Im hier dargestellten Fall kommt es durch die Abkühlung der Flüssigkeit mit Hilfe der Kühlzonen 16 zu einer vertikal gerichteten Abwärtsströmung 5 im äußeren, wandnahen Tankbereich. Die Abwärtsströmung 5 und die höheren Temperaturen in der Tankmitte führen zu einer vertikal gerichteten Aufwärtsströmung 4 (Kernströmung) im Tankinneren bzw. im Leitrohr 36. Das Leitrohr 36 ist gemäß der weiteren Ausführungsform derart gestaltet, dass im Leitrohr 36 ein weiteres Rohr 37 verläuft, welches im oberen Bereich 37_{O} offen und das z. B. mit der Entnahmeleitung 34 verbunden ist. Durch das weitere Rohr 37 kann somit das Produkt aus dem Leitrohr 36 nach unten hin abgeleitet werden. Das Leitrohr 36 selbst ist in seinem oberen Abschnitt 36_{O} geschlossen. Im oberen Tankbereich 30_{O} ist die Befüllleitung 32 angebracht.

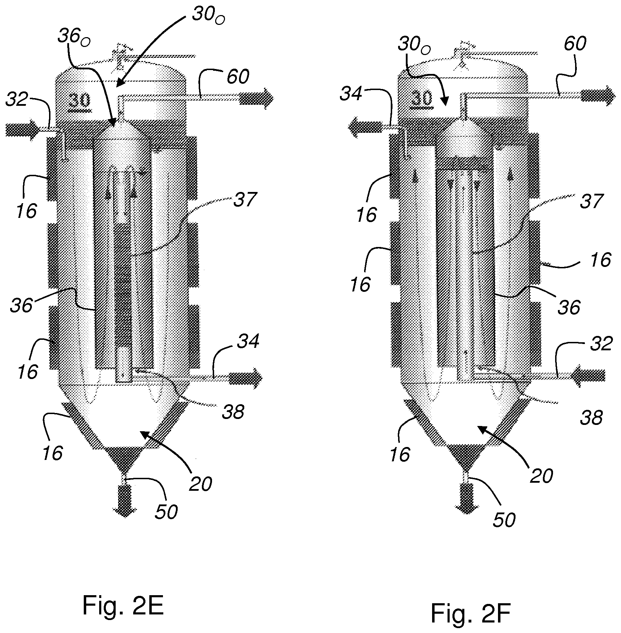

Fig. 2E zeigt vereinfacht eine weitere Ausgestaltung des Tanks 30, wobei das Leitrohr 36 in seinem oberen Abschnitt 36_{O} mit einer Gasableitung 60 verbunden ist. Das Leitrohr 36 ist hier konisch in Richtung des oberen Abschnitts 36_{O} hin verjüngt. Die vorzugsweise zumindest teilweise fermentierte Flüssigkeit im Tank 30 kann hier beispielsweise durch das weitere Rohr 37, welches nach oben hin offen ist 37_{O} und das mit der Entnahmeleitung 34 verbunden ist, abgeführt werden, wobei dabei das Rohr 37 dabei wahlweise auch der Schaumauffangung dienen kann und unterschiedliche Durchmesser und/oder Formen aufweisen kann. Im oberen Tankbereich 30_{O} ist hier beispielsweise die Befüllleitung 32 angebracht.

Fig. 2F zeigt vereinfacht wie der Tank 30 aus Figur 2E bei einer Strömungsumkehrung genutzt werden könnte. In diesem Fall würde die Befüllung über die Befüllleitung 32 in das Rohr 37 erfolgen, welches sich im Leitrohr 36 befindet und nach oben hin offen ist 37_{O} Im oberen Leitrohrabschnitt 36_{O} befindet sich eine zusätzliche Gasableitung 60. Bei entsprechendem Gegendruck fließt hier die Flüssigkeit in einer abwärtsgerichteten Kernströmung 5 im Leitrohr 36 nach unten und steigt im wandnahen Tankbereich in einer Aufwärtsströmung 4 tendenziell in den oberen Tankbereich 30_{O}. In diesem Bereich erfolgt hier vorzugsweise auch der Substratentzug über die Entnahmeleitung 34.

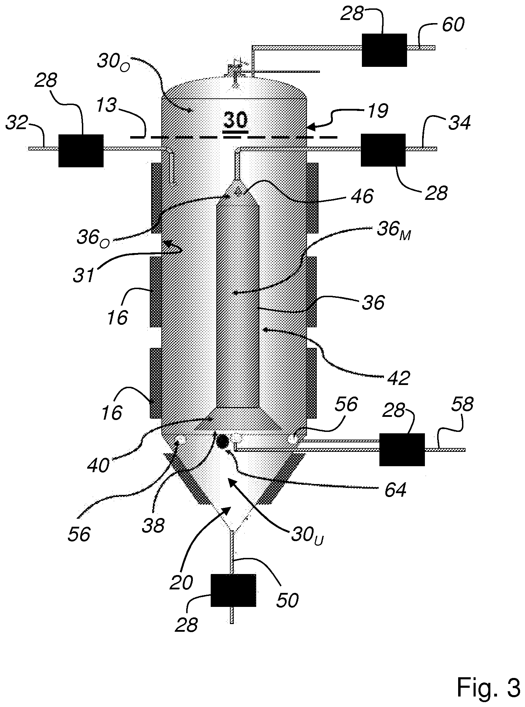

Die schematische Schnittdarstellung der Fig. 3 zeigt eine bevorzugte Variante des modifizierten Tanks 30 zur Fermentation. Wie aus der Darstellung ersichtlich wird, weist der Tank 30 mehrere Kühlzonen 16 auf. Im oberen Tankbereich 30_{O} befindet sich die Befüllleitung 32 und die Entnahmeleitung 34, welche der vorzugsweise kontinuierlichen Zu- und Abführung einer Flüssigkeit (Substrat) dienen und hier eine der Leitungen in ein zentrisch und vertikal installiertes Leitrohr 36 mündet. Die andere Leitung, in der Darstellung nach Fig. 3 die Befüllleitung 32, dient wahlweise dazu, eine im Bezug auf die Tankinnenwand 31 tangential gerichtete Strömung der Flüssigkeit auszubilden. Die Befüllleitung 32 sollte dabei möglichst unterhalb des angestrebten Füllstandniveaus 13 münden. Obwohl in Figur 3 jeweils nur eine Befüllleitung 32 und nur jeweils Entnahmeleitung 34 im oberen Tankbereich (unterhalb des Füllstandniveaus 13) münden, soll dies nicht als eine Beschränkung der Erfindung aufgefasst werden. Die Befüllung und Entleerung des Tanks 30 kann auch in anderen Bereichen erfolgen. Ebenfalls können mehrere Befüllleitungen 32 und Entnahmeleitungen 34 vorgesehen sein, die funktionsgerecht angeordnet sind.

Der obere Abschnitt 36_{O} des Leitrohrs 36 trägt hier einen Verteilschirm 46, wobei wahlweise auch Spritzkugeln o. ä. vorgesehen werden können. Diese Vorrichtungen sollen der Erzeugung einer gleichmäßigen Reinigungsströmung während einer Rückspülung und Reinigung des Leitrohrs 36 und/oder des Tanks 30 dienen. Das Leitrohr 36 kann sich, wie hier dargestellt, bspw. konisch in Richtung des oberen Abschnitts 36_{O} hin verjüngen. Eine Querschnittserweiterung kann jedoch ebenfalls sinnvoll sein, vor allem dann, wenn viel Gas im Leitrohr 36 entsteht, bzw. zugeführt wird. Der mittlere Längsteil 36_{M} des Leitrohrs 36 besteht vorzugsweise aus einem zylindrischen Rohrabschnitt 42, der sich nach unten hin, bevorzugt trichterförmig wie ein Schirm 40, erweitert, wobei das Leitrohr 36 zu seiner Unterseite 38 hin offen ist. Das Leitrohr kann wahlweise in unterschiedlichen Tankhöhen angebracht werden und unterschiedlich lang sein. So kann es im Extremfall unterhalb des Füllstandsniveaus 13 installiert werden, oder wahlweise sogar aus dem Tank 30 nach oben hin hinaus geführt werden. Vorzugsweise sollte es jedoch derart angebracht sein, dass sich der obere Teil zwar oberhalb des Füllstandsniveaus 13, jedoch immer noch innerhalb des Tanks befindet. Im unteren Bereich des Tanks 30 können diverse Elemente zur zusätzlichen Temperierung und/oder zur Gas/Fluid- und/oder Partikelein- bzw. - ausleitung vorgesehen werden. In der Abbildung der Fig. 3 sind daher eine beispielhafte Gaseinleitung 58 sowie eine Heizung/Kühlung 64 dargestellt. Dabei wird vorzugsweise das Gas (beispielsweise Fermentationsgas aus einem vor- oder nachgeschalteten erfindungsgemäßen Tank 30 zur Fermentation) über ein manuell oder automatisch gesteuertes Ventil-, Dosage- und/oder Verteilsystem 28, an dem sich wahlweise Dosagevorrichtungen und/oder Entnahmevorrichtungen befinden können, über die im Tank 30 angebrachten Elemente 56 verteilt. Über eine Gasableitung 60 wird das überschüssige Gas aus dem Tank 30 abgezogen, wobei eine zusätzliche Gasableitung im Leitrohr 36 ebenfalls sinnvoll seien kann. Bevorzugt dienen die Elemente 56 ebenfalls zur Reinigung des Tanks 30. Die Elemente 56 können beispielsweise mittig unterhalb des Leitrohrs 36 oder im äußeren Bereich des Tanks 30 angebracht werden. Der Bodenbereich des Tanks 30 ist in einer bevorzugten Ausführung konisch gestaltet und hat somit eine Konusspitze 20. Die Konusspitze 20 ist mit einer Auslaufleitung 50 zur kontinuierlichen oder chargenweisen Abführung von Partikeln, Stoffen und/oder Organismen verbunden. Zudem ist in der Auslaufleitung 50 ein entsprechendes Ventil-, Dosage- und/oder Verteilsystem 28 vorgesehen, welches automatisch oder manuell gesteuert wird. Damit können die abgeführten Inhaltsstoffe variabel und kontinuierlich oder chargenweise abgezogen werden. Bei Bedarf können diese Stoffe (z. B. Organismen) dann einem vor- und/oder nachgeschalteten Tank 30 zugeführt werden. Dies geschieht vorteilhafterweise über das entsprechenden Ventil-, Dosage- und/oder Verteilsystem 28 des jeweiligen Tankes. Auch die Befüllleitung 32 und/oder die Entnahmeleitung 34 sind vorzugsweise ebenfalls zentral über ein Ventil-, Dosage und/oder Verteilsystem 28 mit entsprechend benötigten Produktförderpumpen, Dosiermöglichkeiten, Verteilventilen, Ent- und Begasungseinheiten, Partikelabscheidern usw. versehen. Dabei sei erwähnt, dass alle in Fig. 3 dargestellten schwarzen Kästen derartige Pumpen, Ventile, Mess-, Regel- und/oder Dosiervorrichtungen u. a. symbolisieren, an denen wahlweise auch eine Entnahme oder Einleitung bestimmter Stoffe erfolgen kann.

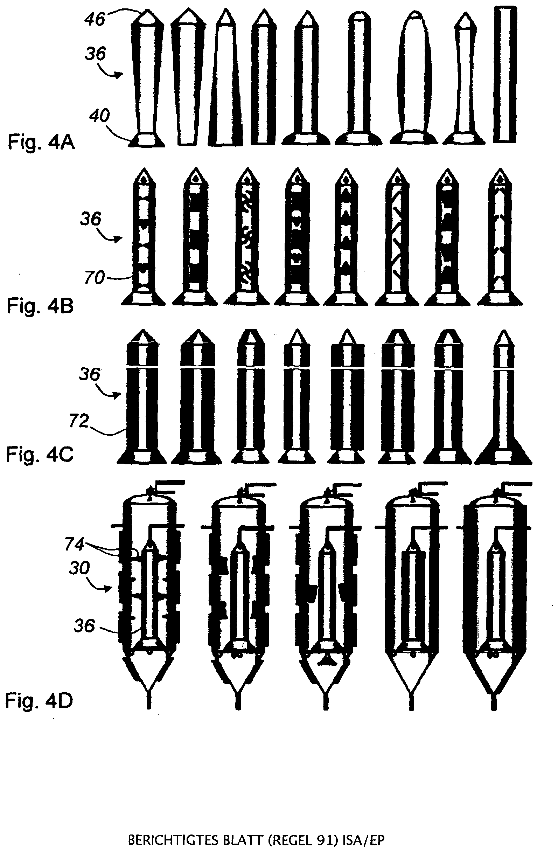

Fig. 4 illustriert vereinfacht einige mögliche Modifikationen der einzelnen Bestandteile des Tanks 30.

So verdeutlicht Fig. 4A vereinfacht mögliche Gestaltungsformen des Leitrohrs 36, das wahlweise zylindrisch und/oder leicht konisch ist und das sich nach unten und/oder nach oben hin wahlweise erweitert und/oder verjüngt. Prinzipiell kann das Leitrohr jedoch beliebig geformt sein und über seine Länge auch unterschiedliche Durchmesser aufweisen. Zudem können die Länge und die Installationshöhe im Tank 30 variieren. Obgleich das Leitrohr möglichst zentral zur Mittelachse 11 hin angebracht werden sollte (vgl. Figur 11), soll eine dezentrale Installation nicht ausgeschlossen werden.

Die Fig. 4B zeigt vereinfacht verschiedene beispielhafte Einbauten 70 im Inneren des Leitrohrs 36 wie Partikel abscheidende, strömungsleitende bzw. Partikel lenkende Einbauten wie Leitbleche, Flügelräder, längs eingebaute Rohrabschnitte etc. Bei der Vorsehung derartiger Installationen sollte jedoch immer darauf geachtet werden, dass eine Reinigung gewährleistet sein muss.

Die Fig. 4C zeigt vereinfacht verschiedene Möglichkeiten, das Leitrohr 36 mit einer zusätzlichen Ummantelung bzw. Isolationen und/oder Temperiervorrichtungen 72 zu versehen. Die Ummantelung 72 kann, wie erwähnt, wahlweise als Heiz- oder Kühlmantel ausgebildet sein, der wahlweise zusätzlich oder ausschließlich noch eine Isolation trägt. Das Leitrohr 36 kann somit zusätzlich oder ausschließlich der Temperierung des Tanks 30 dienen, wobei dieser dann ggf. isoliert sein sollte. Die Anbringung verschiedener Kühlzonen und/oder isolierter Bereiche kann zudem zur Lenkung und Unterstützung der Konvektionsströmungen dienen.

Die schematischen Darstellungen der Fig. 4D zeigen vereinfacht verschiedene Einbauten 74, die neben dem Leitrohr 36 innerhalb des Tanks 30 angeordnet sind, um die Strömung in gewünschter Weise zu lenken und zu beeinflussen.

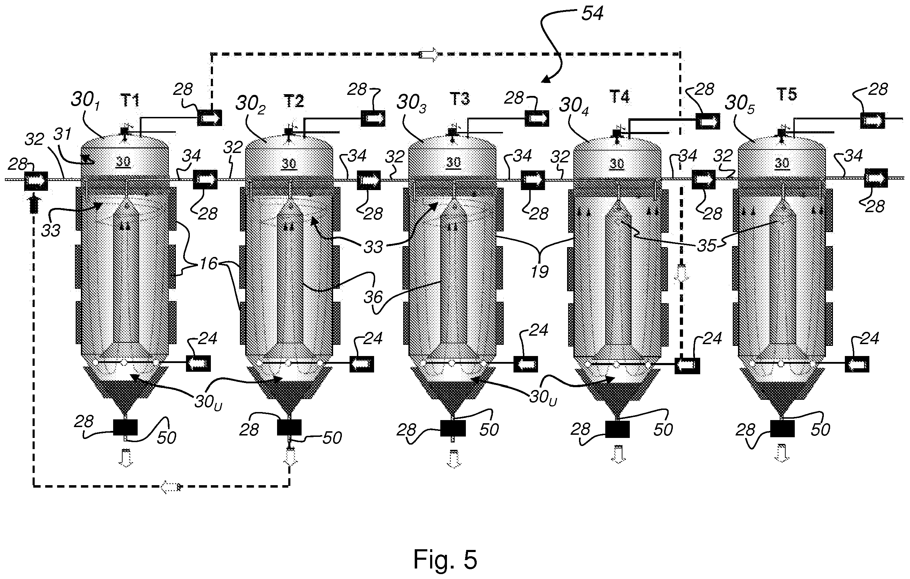

Die schematische Darstellung der Fig. 5 zeigt eine erfindungsgemäße, mehrstufige Anordnung 54 mit fünf nacheinander angeordneten Tanks 30₁, 30₂, 30₃, 30₄ und 30₅ zur Fermentation zur Erzielung eines vorzugsweise kontinuierlichen Fermentationsprozesses, der, wie hier dargestellt, in fünf Prozessschritten T1, T2, T3, T4 und T5 abläuft. Die in Fig. 5 beispielhaft illustrierte mehrstufige Anordnung 54 sieht insgesamt fünf miteinander in einer Kaskade gekoppelte Tanks 30₁, 30₂, 30₃, 30₄ und 30₅ zur Fermentation gemäß Fig. 3 vor, wobei die Strömungsrichtung der beiden letzten Tanks 30₄ und 30₅ gemäß Figur 2C in umgekehrter Richtung geschaltet sind (die Tankbefüllung erfolgt bei diesen Tanks 30₄ und 30₅ nicht über den äußeren Mantelbereich, sondern über das Leitrohr 36). Die auf den Befüllleitungen 32 und den Entnahmeleitungen 34 sowie den Auslaufleitungen 50 dargestellten schwarzen Kästen (Leitungsstellen 28) symbolisieren Ventil-, Dosage- und/oder Verteilsysteme, welche weitere Armaturen wie Pumpen, Abscheidungsvorrichtungen, Mess- und/oder Regelvorrichtungen etc. beinhalten können. Diese Vorrichtungen sollten idealerweise zentral zusammengefasst werden. Wie der Fig. 5 zu entnehmen ist, erfolgt die Befüllung der ersten drei Tanks 30₁, 30₂ und 30₃ jeweils in ihrem oberen Bereich 30_{O} (Bezugszeichen 30_{O} siehe bspw. Fig. 3). Die Befüllleitung 32 ist hier derart angeordnet, dass sich eine im Bezug auf die Tankinnenwand 31 tangential gerichtete Strömung 33 der Flüssigkeit ausbildet. Die Befüllleitung 32 mündet unterhalb des angestrebten Füllstandniveaus 13 (Bezugszeichen 13 siehe ebenfalls Fig. 3). Durch die Anordnung der Leitrohre 36 in den Tanks 30₁, 30₂ und 30₃ und der Kühlzonen 16 kann sich die Flüssigkeit während der Hauptgärung in T1, T2 und T3 jeweils entlang der sich vertikal erstreckenden Längsrichtung der äußeren Mantelbereiche der Tanks 30₁, 30₂ und 30₃ abkühlen und sinkt dabei nach unten. Somit können sich in der Flüssigkeit enthaltene Partikel und/oder Organismen 22 (siehe bspw. Fig. 1) jeweils im unteren Bereich des Tanks 30₁, 30₂ und 30₃ absetzen und kontinuierlich und/oder zyklisch über die Auslaufleitung 50 abgezogen werden. Hierbei kann es von Vorteil sein, wenn zumindest ein Teil der vom unteren Bereich der Tanks 30₁, bis 30₅ abgezogenen Partikel und/oder Organismen 22 eines Tanks 30 - hier bspw. des zweiten Tanks 30₂ - einem oder mehreren der vor oder nach dem betreffenden Tank angeordneten Tank(s) - hier dem vorstehenden Tank 30₁ - über ein entsprechendes Ventil-, Dosage-, und/oder Verteilsystem 28 zugeführt wird. Der Kernstrom der Würze wird dagegen bei der Hauptgärung T1, T2 und T3 durch das in dem jeweiligen Tank 30₁, 30₂ und 30₃ befindliche Leitrohr 36 nach oben hin abgezogen und in den nächsten Tank 30₂, 30₃ und 30₄ geleitet. Um diesen Vorgang zu unterstützen und/oder das Produkt zu verändern, kann zusätzlich Gas und/oder ein anderes Fluid oder Partikel entweder von unten in das jeweilige Leitrohr 36 eingeführt werden, oder über eine Ventilschaltung 28 zugeführt werden. Die beiden letzten Tanks 30₄ und 30₅ stellen in Fig. 5 den Reife- und Lagerprozess T4 und T5 dar. Weil es hier aufgrund der kalten Temperaturen zu einer natürlichen Strömungsumkehrung kommen kann, erfolgt die Einleitung der zugeführten Flüssigkeit in diesem Fall über die entsprechenden Leitrohre 36. Das kälter werdende Produkt steigt hier an der Wandung 19 der jeweiligen Tanks 30₄ und 30₅ auf und wird anschließend in das Leitrohr 36 des nächsten Tanks 30₅ eingeführt bzw. abgeführt. Durch mehrere solcher hintereinander ablaufender Prozessschritte T1 bis T5 kann das Produkt (die Flüssigkeit) immer weiter abgekühlt werden und parallel dazu können idealerweise immer mehr (kleinere) Partikel 22 abgeschieden werden. Das fertige, vorzugsweise vorgeklärte Produkt wird hier aus dem letzten Tank 30₅ von oben abgezogen. Ein kontinuierlicher Produktstrom kann entweder über eine entsprechende Druckregulation oder über Förderpumpen, die sich beispielsweise in den Entnahmeleitungen 34 an Ventil-Dosage- und/oder Verteilsystemen 28 befinden, gewährleistet werden. Bei der druckgesteuerten Förderung sollte der Behälterdruck des vorgeschalteten Tanks 30₁, 30₂, 30₃, 30₄ zur Fermentation größer sein als der Druck des jeweils nachgeschalteten Tanks 30₂, 30₃, 30₄, 30₅. Beispielsweise sollte der Druck des Tanks 30₁ größer als der Druck des Tanks 30₂ sein. Der Druck im Tank 30₂ sollte wiederum höher sein als der Druck im nachgeschalteten Tank 30₃ usw.. Auf diese Weise kann die Förderrichtung für die Flüssigkeit definiert werden.

Walhlweise oder zusätzlich kann eine Temperierung, wie z.B. eine gezielte Erwärmung und/oder Abkühlung, dazu genutzt werden die biochemischen und/oder physikalischen Prozesse auf gewünschte Art und Weise zu beeinflussen. Zu diesem Zweck können wahlweise an einigen oder an allen Ventil-Dosage- und/oder Verteilsystemen 28 zusätzlich oder ausschließlich derartige Temperiervorrichtungen, wie z.B. Plattenwärmetauscher, Rohrbündelwärmetauscher und/oder Kurzzeiterhitzer vorgesehen werden. So kann beispielsweise eine gezielte Erhitzung des Produktes z. B. den Diacetylabbau im späteren Prozessverlauf erleichtern und/oder eine mikrobiologische Sicherheit gewährleisten.

Mit der gezeigten mehrstufigen Anordnung 54 werden Getränke hergestellt. Wie hier beschrieben, eignet sich die Vorrichtung zur Erzeugung von Bioethanol oder zur Produktion von Methangas. Sie kann zur Verarbeitung von Molke und/oder zur Herstellung von Essigsäure oder zur Herstellung von Milchsäure und/oder Sojasoße dienen.

### Bezugszeichenliste

- 4: Aufwärtsströmung
- 5: Abwärtsströmung
- 8: abgezogene Hefe
- 10: Basisfermentat, Substrat
- 11: Mittelachse
- 12: Tank (Stand der Technik)
- 12_{U}: Unterer Tankbereich
- 13: Füllstandniveau
- 14: Hefewiederverwendung (Inokulat)
- 15: Reinigungsanschluss
- 16: Kühlzone
- 17: Domarmatur mit Entgasungsvorrichtung
- 18: Konvektionsströmung
- 19: Wandung
- 20: Konusspitze
- 22: Sedimentierte Hefe (Partikel)
- 28: Ventil-, Dosage-, und/oder Verteilsystem
- 30: Erfindungsgemäßer Tank zur Fermentation
- 30₁: Erster kaskadierter Tank
- 30₂: Zweiter kaskadierter Tank
- 30₃: Dritter kaskadierter ank
- 30₄: Vierter kaskadierter Tank
- 30₅: Fünfter kaskadierter Tank
- 30_{O}: Oberer Bereich des jeweiligen Tanks
- 30_{U}: Unterer Bereich des jeweiligen Tanks
- 31: Innenwand des jeweiligen Tanks
- 32: Befüllleitung
- 33: Tangentiale Strömung
- 34: Entnahmeleitung
- 36: Leitrohr
- 36_{M}: mittleres Längsteil des Leitrohrs
- 36_{O}: Oberer Abschnitt der Leitrohrs
- 37: Rohr
- 37_{O}: Offener Rohrabschnitt
- 38: Offene Unterseite des Leitrohrs
- 40: Schirm
- 42: Zylindrischer Rohrabschnitt
- 46: Verteilschirm / Spritzkugel u. ä.
- 50: Auslaufleitung
- 54: Mehrstufige Anordnung
- 56: Elemente zur Gaseinleitung
- 58: Gaseinleitung
- 60: Zusätzliche Gasableitung des leitrohres
- 64: Heizung/Kühlung
- 70: Einbauten im Leitrohr
- 72: Ummantelung/Temperierung/Isolation
- 74: Einbauten im Tank

## Patentansprüche

1. Vorrichtung zur Fermentation aus einer mehrstufigen Anordnung (54) von zwei oder mehreren, miteinander in einer Kaskade gekoppelten Tanks (30₁, 30₂, 30₃, 30₄, 30₅) zur Fermentation einer Flüssigkeit zur Herstellung eines Getränks, bei der mindestens eine Entnahmeleitung (34) eines ersten Tanks (30₁) zur Fermentation mit mindestens einer Befüllleitung (32) eines zweiten Tanks (30₂) zur Fermentation verbunden ist, dessen mindestens eine Entnahmeleitung (34) zu mindestens einer weiteren Befüllleitung (32) eines nachgeordneten dritten Tanks (30₃) zur Fermentation führt, und wobei den Tanks (30₁, 30₂, 30₃, 30₄, 30₅) Kühlzonen (16) zugeordnet sind, welche wahlweise auch/oder ausschließlich an den Leitrohren angebracht sein können, so dass die Tanks (30₁, 30₂, 30₃, 30₄, 30₅) jeweils unterschiedlich und/oder individuell temperierbar sind; und wobei
jeder der Tanks (30, 30₁, 30₂, 30₃, 30₄, 30₅) mindestens eine Befüllleitung (32) zur Zuführung der zu fermentierenden Flüssigkeit und mindestens eine Entnahmeleitung (34) zur Ableitung einer zumindest teilweise fermentierten Flüssigkeit aufweist,
jeder der Tanks (30, 30₁, 30₂, 30₃, 30₄, 30₅) zur Fermentation temperierbar ist,
jeder der Tanks (3) zur Fermentation eine Auslaufleitung (50) im unteren, sich trichterförmig verjüngenden Bereich (30_{U}) des Tanks (30, 30₁, 30₂, 30₃, 30₄, 30₅) aufweist,
in jedem der Tanks (30, 30₁, 30₂, 30₃, 30₄, 30₅) ein Leitrohr (36) sich innerhalb des Tanks (30, 30₁, 30₂, 30₃, 30₄, 30₅) zur Fermentation erstreckt, wobei das Leitrohr (36) zur Lenkung und/oder Beruhigung der im Tank (30, 30₁, 30₂, 30₃, 30₄, 30₅) befindlichenFlüssigkeiten dient und nicht mit der Auslaufleitung (50) in Verbindung steht, und
in jedem der Tanks (30, 30₁, 30₂, 30₃, 30₄, 30₅) die mindestens eine Befüllleitung (32) oder mindestens eine Entnahmeleitung (34) mit dem Leitrohr (36) verbunden ist und das Leitrohr (36) eine offene Unterseite (38) aufweist, so dass ein kontinuierlicher Zu- und Abfluss von Substrat bzw. zumindest teilweise fermentierter Flüssigkeit gewährleistet ist; und
wobei die Auslaufleitung (50) im Bodenbereich zumindest eines der Tanks (30₁, 30₂, 30₃, 30₄, 30₅) mit der mindestens einen Befüllleitung (32) eines anderen vor und/oder nachgeschalteten Tanks (30₁, 30₂, 30₃, 30₄, 30₅) verbunden ist.

2. Vorrichtung nach Anspruch 1, wobei in jedem der Tanks (30, 30₁, 30₂, 30₃, 30₄, 30₅) das Leitrohr (36) in einem oberen Bereich (36ₒ) einen konischen Verteilschirm (46) trägt und die mindestens eine Befüllleitung (32) oder die mindestens eine Entnahmeleitung (34) mit einem oberen Bereich (30ₒ) des Tanks (30) verbunden sind und unterhalb eines maximalen Füllstandniveaus (13) münden.

3. Vorrichtung nach einem der Ansprüche 1 bis 2, wobei in jedem der Tanks (30, 30₁, 30₂, 30₃, 30₄, 30₅) das Leitrohr (36) strömungslenkende und/oder sedimentationsfördernde Einbauten (70) aufweist.

4. Vorrichtung nach einem der Ansprüche 1 bis 3, wobei in jedem der Tanks (30, 30₁, 30₂, 30₃, 30₄, 30₅) das Leitrohr (36) im oberen Abschnitt (36O) geschlossen ist und im Leitrohr (36) mindestens ein weiteres Rohr (37) vorgesehen ist, wobei die mindestens eine Befüllleitung (32) oder die mindestens eine Entnahmeleitung (34) mit einem der weiteren Rohre (37) verbunden ist und wobei eine Gasableitung (60) am oberen Abschnitt (36ₒ) des Leitrohrs (36) angebracht ist.

5. Vorrichtung nach einem der Ansprüche 1 bis 4, wobei in jedem der Tanks (30, 30₁, 30₂, 30₃, 30₄, 30₅) das Leitrohr (36) zumindest teilweise mit einer Ummantelung (72) versehen und dadurch temperierbar und/oder thermisch isoliert ist.

6. Vorrichtung nach einem der vorhergehenden Ansprüche, wobei ein Druck in einem vorgeschalteten Tank (30₁, 30₂, 30₃, 30₄) zur Fermentation größer ist als ein Druck eines jeweils unmittelbar nachgeschalteten Tanks (30₂, 30₃, 30₄, 30₅) zur Fermentation.

7. Vorrichtung nach einem der vorhergehenden Ansprüche, wobei in mindestens einer Auslaufleitung (50) eine Ventil-, Dosage-, und/oder Verteilvorrichtung (28) in Form einer Förderpumpe zwischen zumindest zwei aufeinander folgenden Tanks (30₁, 30₂, 30₃, 30₄, 30₅) zur Fermentation angeordnet ist, und zumindest eine Gasableitung (60) mit einer Gaseinleitung (58) der vor und/oder nachgeschalteten Tanks (30₁, 30₂, 30₃, 30₄, 30₅) verbunden ist.

8. Vorrichtung gemäß einem der vorhergehenden Ansprüche, wobei in jedem der Tanks (30₁, 30₂, 30₃, 30₄, 30₅) das Leitrohr (36) einen Durchmesser aufweist, der wenigstens 15% und weniger als 70% des Durchmessers des Tanks (30) entspricht.

9. Verfahren zur kontinuierlichen oder semikontinuierlichen Fermentation von Flüssigkeiten zur Herstellung von Getränken innerhalb einer Vorrichtung gemäß einem der Ansprüche 1 bis 8, mit den folgenden Schritten:
• dass in mindestens einem der Tanks (30₁, 30₂, 30₃, 30₄, 30₅) die zu fermentierende Flüssigkeit über mindestens eine Befüllleitung (32) in einen oberen Bereich (30ₒ) des mindestens einen der Tanks (30₁, 30₂, 30₃, 30₄, 30₅) zur Fermentation eingeleitet wird,
• dass mittels eines sich innerhalb des mindestens einen der Tanks (30₁, 30₂, 30₃, 30₄, 30₅) erstreckenden Leitrohrs (36) die Flüssigkeit gelenkt und/oder beruhigt wird,
• dass die Flüssigkeit zu mindestens einer Entnahmeleitung (34) geleitet wird, in die das Leitrohr (36) mündet; und
dass sich in der Flüssigkeit enthaltene Partikel und/oder Organismen in einem unteren, sich trichterförmig verjüngenden Bereich (20_{U}) des mindestens einen der Tanks (30₁, 30₂, 30₃, 30₄, 30₅) absetzen und kontinuierlich und/oder zyklisch über eine Auslaufleitung (50), die nicht mit dem Leitrohr (36) in Verbindung steht, aus einem der Tanks (30₁, 30₂, 30₃, 30₄, 30₅) abgezogen werden.

10. Verfahren nach Anspruch 9, wobei die zu fermentierende Flüssigkeit über die mindestens eine Befüllleitung (32) über eine Wandung (19) des mindestens einen der Tanks (30₁, 30₂, 30₃, 30₄, 30₅) oder über die mindestens eine Befüllleitung (32) von oben in das Leitrohr (36) eingeleitet wird.

11. Verfahren nach einem der Ansprüche 9 bis 10, wobei am mindestens einen der Tanks (30₁, 30₂, 30₃, 30₄, 30₅) Kühlzonen (16) angebracht sind, die derart gesteuert werden, dass sich die Flüssigkeit entlang der Wandung (19) des mindestens einen der Tanks (30₁, 30₂, 30₃, 30₄, 30₅) von oben nach unten abkühlt und dabei eine Abwärtsströmung (5) ausgebildet wird, oder dass die Flüssigkeit entlang der Wandung (19) des mindestens einen der Tanks (30₁, 30₂, 30₃, 30₄, 30₅) von unten nach oben abkühlt und dabei eine Aufwärtsströmung (4) ausgebildet wird.

12. Verfahren nach einem der Ansprüche 9 bis 11, wobei zumindest ein Teil der vom unteren Bereich (20_{U}) abgezogenen Partikel und/oder Organismen eines der Tanks (30₁, 30₂, 30₃, 30₄, 30₅) einem oder mehreren der vor und/oder nach dem jeweiligen Tank (30₁, 30₂, 30₃, 30₄, 30₅) angeordneten Tank (30₁, 30₂, 30₃, 30₄, 30₅) wieder zugeführt wird.

13. Verfahren nach Anspruch 12, wobei die Flüssigkeit in aufeinander folgenden Tanks (30₁, 30₂, 30₃, 30₄, 30₅) der Tanks (30₁, 30₂, 30₃, 30₄, 30₅) zunehmend abgekühlt und aus ihr zunehmend Partikel und/oder Organismen abgezogen werden.

14. Verfahren nach einem der Ansprüche 9 bis 13, wobei über mindestens eine Befüllleitung (32) eines der Tanks (30₁, 30₂, 30₃, 30₄, 30₅) wenigstens ein Zusatzstoff eindosiert wird und/oder über wenigstens eine Gaseinleitung (58) in einem unteren Bereich (30_{U}) des einen der Tanks (30₁, 30₂, 30₃, 30₄, 30₅) ein Fermentationsgas und/oder andere Gase eingeleitet werden.

## Claims

1. Apparatus for fermentation, comprising a multi-stage arrangement (54) of two or more tanks (30₁, 30₂, 30₃, 30₄, 30₅), coupled to one another in a cascade, for fermenting a liquid in order to produce a beverage, in which arrangement at least one removal line (34) of a first tank (30₁) for fermentation is connected to at least one filling line (32) of a second tank (30₂) for fermentation, the at least one removal line (34) of which second tank leads to at least one further filling line (32) of a third tank (30₃) for fermentation that is arranged downstream, and wherein cooling regions (16) are assigned to the tanks (30₁, 30₂, 30₃, 30₄, 30₅), which cooling regions can be selectively also/or exclusively attached to the guide pipes such that the temperature of each of the tanks (30₁, 30₂, 30₃, 30₄, 30₅) can be differently and/or individually controlled; and wherein
each of the tanks (30, 30₁, 30₂, 30₃, 30₄, 30₅) comprises at least one filling line (32) for supplying the liquid to be fermented and at least one removal line (34) for conducting away an at least partially fermented liquid,
the temperature of each of the tanks (30, 30₁, 30₂, 30₃, 30₄, 30₅) for fermentation can be controlled,
each of the tanks (3) for fermentation comprises an outlet line (50) in the lower region (30ᵤ) of the tank (30, 30₁, 30₂, 30₃, 30₄, 30₅), which lower region tapers in the manner of a funnel,
a guide pipe (36) extends in each of the tanks (30, 30₁, 30₂, 30₃, 30₄, 30₅) inside the tank (30, 30₁, 30₂, 30₃, 30₄, 30₅) for fermentation, wherein the guide pipe (36) is intended for deflecting and/or calming the liquids located in the tank (30, 30₁, 30₂, 30₃, 30₄, 30₅) and is not connected to the outlet line (50), and
in each of the tanks (30, 30₁, 30₂, 30₃, 30₄, 30₅) the at least one filling line (32) or at least one removal line (34) is connected to the guide pipe (36) and the guide pipe (36) is open at the bottom (38) such that a continuous inflow and outflow of substrate or at least partially fermented liquid is ensured; and wherein the outlet line (50) in the bottom region of at least one of the tanks (30₁, 30₂, 30₃, 30₄, 30₅) is connected to the at least one filling line (32) of a different tank (30₁, 30₂, 30₃, 30₄, 30₅) that is connected upstream and/or downstream.

2. Apparatus according to claim 1, wherein in each of the tanks (30, 30₁, 30₂, 30₃, 30₄, 30₅) the guide pipe (36) carries a conical distribution screen (46) in an upper region (36ₒ) and the at least one filling line (32) or the at least one removal line (34) is connected to an upper region (30ₒ) of the tank (30) and opens out below a maximum fill level (13).

3. Apparatus according to either of claims 1 to 2, wherein in each of the tanks (30, 30₁, 30₂, 30₃, 30₄, 30₅) the guide pipe (36) comprises built-in elements (70) for deflecting the flow and/or conveying sediment.

4. Apparatus according to any of claims 1 to 3, wherein in each of the tanks (30, 30₁, 30₂, 30₃, 30₄, 30₅) the guide pipe (36) is closed in the upper portion (36O) and at least one additional pipe (37) is provided in the guide pipe (36), wherein the at least one filling line (32) or the at least one removal line (34) is connected to one of the additional pipes (37) and wherein a gas discharge (60) is attached to the upper portion (36ₒ) of the guide pipe (36).

5. Apparatus according to any of claims 1 to 4, wherein in each of the tanks (30, 30₁, 30₂, 30₃, 30₄, 30₅) the guide pipe (36) is provided at least in part with a casing (72) and can be temperature controlled and/or is thermally insulated thereby.

6. Apparatus according to any of the preceding claims, wherein a pressure in a tank (30₁, 30₂, 30₃, 30₄) for fermentation connected upstream is greater than a pressure of a tank (30₂, 30₃, 30₄, 30₅) for fermentation connected respectively directly downstream.

7. Apparatus according to any of the preceding claims, wherein a valve device, metering device and/or distributing device (28), in the form of a feed pump, is arranged between at least two successive tanks (30₁, 30₂, 30₃, 30₄, 30₅) for fermentation in at least one outlet line (50), and at least one gas discharge (60) is connected to a gas inlet line (58) of the tank (30₁, 30₂, 30₃, 30₄, 30₅) connected upstream and/or downstream.

8. Apparatus according to any of the preceding claims, wherein in each of the tanks (30₁, 30₂, 30₃, 30₄, 30₅) the guide pipe (36) has a diameter that corresponds to at least 15% and less than 70% of the diameter of the tank (30).

9. Method for continuous or semi-continuous fermentation of liquids in order to produce beverages inside an apparatus according to any of claims 1 to 8, said method comprising the following steps:
• whereby in at least one of the tanks (30₁, 30₂, 30₃, 30₄, 30₅) the liquid to be fermented is introduced via at least one filling line (32) into an upper region (30ₒ) of the at least one tank (30₁, 30₂, 30₃, 30₄, 30₅) for fermentation,
• whereby the liquid is deflected and/or calmed by means of a guide pipe (36) that extends inside the at least one tank (30₁, 30₂, 30₃, 30₄, 30₅),
• whereby the liquid is guided to at least one removal line (34) into which the guide pipe (36) opens; and
whereby particles and/or organisms contained in the liquid settle in a lower region (20ᵤ) of at least one of the tanks (30₁, 30₂, 30₃, 30₄, 30₅) that tapers in the manner of a funnel and are continuously and/or cyclically removed from one of the tanks (30₁, 30₂, 30₃, 30₄, 30₅) via an outlet line (50) that is not connected to the guide pipe (36).

10. Method according to claim 9, wherein the liquid to be fermented is introduced into the guide pipe (36) via the at least one filling line (32) via a wall (19) of the at least one tank (30₁, 30₂, 30₃, 30₄, 30₅) or from above via the at least one filling line (32).

11. Method according to any of claims 9 to 10, wherein cooling regions (16) are attached to at least one of the tanks (30₁, 30₂, 30₃, 30₄, 30₅) and are controlled such that the liquid cools down from top to bottom along the wall (19) of the at least one tank (30₁, 30₂, 30₃, 30₄, 30₅) and, in the process, a downward flow (5) is produced, or such that the liquid cools down from bottom to top along the wall (19) of the at least one tank (30₁, 30₂, 30₃, 30₄, 30₅) and, in the process, an upward flow (4) is produced.

12. Method according to any of claims 9 to 11, wherein at least some of the particles and/or organisms of one of the tanks (30₁, 30₂, 30₃, 30₄, 30₅) removed from the lower region (20ᵤ) are fed back to one or more of the tanks (30₁, 30₂, 30₃, 30₄, 30₅) arranged upstream and/or downstream of the tank (30₁, 30₂, 30₃, 30₄, 30₅) in question.

13. Method according to claim 12, wherein the liquid cools down progressively in successive tanks (30₁, 30₂, 30₃, 30₄, 30₅) of the tanks (30₁, 30₂, 30₃, 30₄, 30₅) and an increasing quantity of particles and/or organisms are removed therefrom.

14. Method according to any of claims 9 to 13, wherein at least one additive is metered in via at least one filling line (32) of one of the tanks (30₁, 30₂, 30₃, 30₄, 30₅) and/or a fermentation gas and/or other gases is/are introduced via at least one gas inlet line (58) in a lower region (30ᵤ) of one of the tanks (30₁, 30₂, 30₃, 30₄, 30₅).

## Revendications

1. Dispositif de fermentation consistant en un agencement (54) sur plusieurs étages de deux ou plusieurs réservoirs (30₁, 30₂, 30₃, 30₄, 30₅) accouplés les uns aux autres en cascade pour la fermentation d'un liquide pour la production d'une boisson, dans lequel au moins une conduite de prélèvement (34) d'un premier réservoir (30₁) pour la fermentation est reliée à au moins une conduite de remplissage (32) d'un second réservoir (30₂) pour la fermentation, dont l'au moins une conduite de prélèvement (34) mène à au moins une autre conduite de remplissage (32) d'un troisième réservoir (30₃) pour la fermentation disposé en aval, et où des zones de refroidissement (16), qui peuvent être, au choix, aussi ou exclusivement attachées aux tubes directeurs, sont associées aux réservoirs (30₁, 30₂, 30₃, 30₄, 30₅), de sorte que les réservoirs (30₁, 30₂, 30₃, 30₄, 30₅) peuvent être respectivement tempérés différemment et/ou individuellement ; et où
chacun des réservoirs (30, 30₁, 30₂, 30₃, 30₄, 30₅) comporte au moins une conduite de remplissage (32) pour l'amenée du liquide à faire fermenter et au moins une conduite de prélèvement (34) pour le déversement d'un liquide au moins partiellement fermenté,
chacun des réservoirs (30, 30₁, 30₂, 30₃, 30₄, 30₅) peut être tempéré pour la fermentation, chacun des réservoirs (3) pour la fermentation comporte une conduite d'écoulement (50) dans la région (30ᵤ) inférieure du réservoir (30, 30₁, 30₂, 30₃, 30₄, 30₅) se rétrécissant en forme d'entonnoir,
dans chacun des réservoirs (30, 30₁, 30₂, 30₃, 30₄, 30₅) un tube directeur (36) s'étend à l'intérieur du réservoir (30, 30₁, 30₂, 30₃, 30₄, 30₅) pour la fermentation, le tube directeur (36) servant à guider et/ou calmer le liquide se trouvant dans le réservoir (30, 30₁, 30₂, 30₃, 30₄, 30₅) et n'étant pas relié à la conduite d'écoulement (50), et
dans chacun des réservoirs (30, 30₁, 30₂, 30₃, 30₄, 30₅) l'au moins une conduite de remplissage (32) ou l'au moins une conduite de prélèvement (34) est reliée avec le tube directeur (36) et le tube directeur (36) comporte un côté inférieur (38) ouvert, de manière à assurer un afflux et une évacuation continue de substrat, respectivement de liquide partiellement fermenté ; et
où la conduite d'écoulement (50) est reliée dans la région du fond d'au moins un des réservoirs (30₁, 30₂, 30₃, 30₄, 30₅) avec l'au moins une conduite de remplissage (32) d'un autre réservoir (30₁, 30₂, 30₃, 30₄, 30₅) disposé en amont et/ou disposé en aval.

2. Dispositif selon la revendication 1, où dans chacun des réservoirs (30, 30₁, 30₂, 30₃, 30₄, 30₅) le tube directeur (36) porte dans une région supérieure (36ₒ) un écran de distribution (46) conique et l'au moins une conduite de remplissage (32) ou l'au moins une conduite de prélèvement (34) est reliée avec une région supérieure (30ₒ) du réservoir (30) et débouche sous un niveau de remplissage maximal (13).

3. Dispositif selon l'une des revendications 1 à 2, où dans chacun des réservoirs (30, 30₁, 30₂, 30₃, 30₄, 30₅) le tube directeur (36) comporte des organes (70) guidant l'écoulement et/ou favorisant la sédimentation.

4. Dispositif selon l'une des revendications 1 à 3, où dans chacun des réservoirs (30, 30₁, 30₂, 30₃, 30₄, 30₅), le tube directeur (36) est fermé dans la section supérieure (36ₒ) et au moins un tube supplémentaire (37) est prévu dans le tube directeur (36), où l'au moins une conduite de remplissage (32) ou l'au moins une conduite de prélèvement (34) est reliée avec l'un des tubes supplémentaires (37) et où une évacuation de gaz (60) est attachée à la section supérieure (36ₒ) du tube directeur (36).

5. Dispositif selon l'une des revendications 1 à 4, où dans chacun des réservoirs (30, 30₁, 30₂, 30₃, 30₄, 30₅), le tube directeur (36) est au moins partiellement pourvu d'une gaine (72) et peut ainsi être tempérer ou bien est thermiquement isolé.

6. Dispositif selon l'une des revendications précédentes, où une pression dans un réservoir (30₁, 30₂, 30₃, 30₄, 30₅) pour la fermentation disposé en amont est supérieure à une pression d'un réservoir (30₂, 30₃, 30₄, 30₅) pour la fermentation, disposé respectivement directement en aval.

7. Dispositif selon l'une des revendications précédentes, où dans au moins une conduite d'écoulement (50), un dispositif de soupape, de dosage et/ou de distribution (28) sous la forme d'une pompe de circulation est disposé entre au moins deux réservoirs (30₁, 30₂, 30₃, 30₄, 30₅) pour la fermentation consécutifs, et au moins une évacuation de gaz (60) est reliée avec une introduction de gaz (58) des réservoirs (30₁, 30₂, 30₃, 30₄, 30₅) disposés en aval et/ou disposés en amont.

8. Dispositif selon l'une des revendications précédentes, où dans chacun des réservoirs (30₁, 30₂, 30₃, 30₄, 30₅), le tube directeur (36) comporte un diamètre qui correspond à au moins 15% et moins de 70% du diamètre du réservoir (30).

9. Procédé pour la fermentation continue ou semi-continue de liquides pour la production de boissons à l'intérieur d'un dispositif selon l'une des revendications 1 à 8, comportant les étapes suivantes :
• dans au moins un des réservoirs (30₁, 30₂, 30₃, 30₄, 30₅), le liquide à faire fermenter est introduit par au moins une conduite de remplissage (32) dans une région supérieure (30ₒ) de l'au moins un des réservoirs (30₁, 30₂, 30₃, 30₄, 30₅) pour la fermentation,
• le liquide est guidé et/ou calmé au moyen d'un tube directeur (36) s'étendant à l'intérieur de l'au moins un des réservoirs (30₁, 30₂, 30₃, 30₄, 30₅),
• le liquide est conduit à au moins une conduite de prélèvement (34) dans laquelle débouche le tube directeur (36) ; et
des particules et/ou organismes contenus dans le liquide se déposent dans une région inférieure (20ᵤ) de l'au moins un des réservoirs (30₁, 30₂, 30₃, 30₄, 30₅) se rétrécissant en forme d'entonnoir, et sont retirés continuellement et/ou cycliquement de l'un des réservoirs (30₁, 30₂, 30₃, 30₄, 30₅) par l'intermédiaire d'une conduite d'écoulement (50) qui n'est pas reliée au tube directeur (36).

10. Procédé selon la revendication 9, où le liquide à faire fermenter est introduit dans le tube directeur (36) via l'au moins une conduite de remplissage (32) via une paroi (19) de l'au moins un des réservoirs (30₁, 30₂, 30₃, 30₄, 30₅), ou par en haut via l'au moins une conduite de remplissage (32).

11. Procédé selon l'une des revendications 9 à 10, où des zones de refroidissement (16) sont attachées à l'au moins un des réservoirs (30₁, 30₂, 30₃, 30₄, 30₅), ces zones étant commandées de telle manière que le liquide se refroidit de haut en bas le long de la paroi (19) de l'au moins un des réservoirs (30₁, 30₂, 30₃, 30₄, 30₅) formant ainsi un courant vers le bas (5), ou que le liquide se refroidit de bas en haut le long de la paroi (19) de l'au moins un des réservoirs (30₁, 30₂, 30₃, 30₄, 30₅) formant ainsi un courant vers le haut (4).

12. Procédé selon l'une des revendications 9 à 10, où au moins une partie des particules et/ou organismes de l'un des réservoirs (30₁, 30₂, 30₃, 30₄, 30₅) prélevés dans la partie inférieure (20ᵤ) est réintroduite dans un ou plusieurs des réservoirs (30₁, 30₂, 30₃, 30₄, 30₅) disposés avant et/ou après le réservoir (30₁, 30₂, 30₃, 30₄, 30₅) respectif.

13. Procédé selon la revendication 12, où le liquide dans des réservoirs (30₁, 30₂, 30₃, 30₄, 30₅) successifs parmi les réservoirs (30₁, 30₂, 30₃, 30₄, 30₅) est de plus en plus refroidit et des particules et/ou organismes en sont prélevés de manière croissante.

14. Procédé selon l'une des revendications 9 à 13, où au moins un additif est introduit de manière dosée via au moins une conduite de remplissage (32) de l'un des réservoirs (30₁, 30₂, 30₃, 30₄, 30₅), et/ou un gaz de fermentation et/ou d'autres gaz sont introduits via au moins une introduction de gaz (58) dans une région inférieure (30ᵤ) de ce réservoir (30₁, 30₂, 30₃, 30₄, 30₅).
